# EUROPÄISCHE PATENTANMELDUNG

(11) **EP 2 014 662 A1**
(43) Veröffentlichungstag der Anmeldung: **14.01.2009**
(21) Anmeldenummer: 07075602.8
(22) Anmeldetag: 12.07.2007
(51) Int. Cl.: C07D 495/04, A61K 31/519

(54) **Indolylalkylthienopyrimidylamine als Modulatoren des EP2-Rezeptors**

(71) Anmelder: Bayer Schering Pharma Aktiengesellschaft, 13353 Berlin (DE)
(72) Erfinder: Buchmann, Bernd, 16540 Hohen Neuendorf (DE); Bräeuer, Nico, 07743 Jena (DE); Koppitz, Marcus, 10115 Berlin (DE); Peters, Olaf, 99891 Tabarz (DE); Ter Laak, Antonius, 12159 Berlin (DE); Lindenthal, Bernhard, 13507 Berlin (DE); Langer, Gernot, 14612 Falkensee (DE); Wintermantel, Tim, 10178 Berlin (DE)

(57) **Zusammenfassung**

Die vorliegende Erfindung betrifft Indolylalkylthienopyrimidylamine der allgemeinen Formel I, Verfahren zu ihrer Herstellung sowie deren Verwendung zur Herstellung von pharmazeutischen Mitteln zur Behandlung von Erkrankungen und Indikationen, die im Zusammenhang stehen mit dem EP₂ Rezeptor.

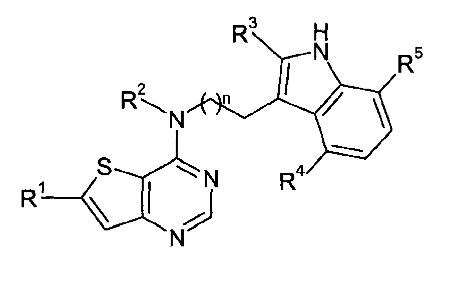

## Beschreibung

Es ist schon lange bekannt, dass Prostaglandine Schlüsselmoleküle bei den Prozessen der weiblichen Reproduktionsbiologie, wie z. B. der Regulation der Ovulation, der Fertilisation, der Nidation, der Dezidualisierung (z.B. Plazentabildung) und der Menstruation, sind. Prostaglandine spielen ebenfalls eine wichtige Rolle bei den pathologischen Veränderungen im reproduktiven Trakt, einschließlich der Menorrhagie, Dysmenorrhoe, Endometriose und Krebs. Bisher ist der Mechanismus, durch den Prostaglandine diese Veränderungen bewirken, noch nicht vollständig geklärt. Neuere Erkenntnisse weisen darauf hin, dass Prostaglandine, ihre Rezeptoren und deren Signaltransduktionswege an Prozessen wie Angiogenese, Apoptose, Proliferation sowie an inflammatorischen/antiinflammatorischen und immunologischen Prozessen beteiligt sind.

Die Wirkungen der Prostaglandine werden durch ihre G-Protein-gekoppelten Rezeptoren, die auf der Zelloberfläche lokalisiert sind, vermittelt. Von besonderem Interesse ist das Prostaglandin E₂ (PGE₂), das unterschiedlichste zelluläre Wirkungen erzielt, indem es an funktionell verschiedene Rezeptorensubtypen, nämlich die EP₁, EP₂, EP₃ und EP₄ Rezeptoren, bindet. Die Rezeptor-Subtypen, an denen das Prostaglandin E₂ bindet, scheinen für die Rezeptor vermittelten Wirkungen, die bei der Fertilitätsregulation eine Rolle spielen, von besonderem Interesse zu sein. So konnte gezeigt werden, dass die reproduktiven Funktionen bei EP₂ Knock-out Mäusen (EP₂ ^{-/-}), d.h. bei Mäusen, die keinen funktionstüchtigen PGE₂-Rezeptor Subtyp EP₂ mehr besitzen, beeinträchtigt sind, und dass diese Tiere eine kleinere "Wurfanzahl" haben (Matsumoto et al., 2001, Biology of Reproduction 64, 1557-1565). Ebenso konnte gezeigt werden, dass diese EP₂ Knock-out Mäuse (Hizaki et al. Proc Natl Acad Sci U. S. A. 1999 Aug 31; 96(18):10501-10506) eine deutlich verminderte Kumulus Expansion und starke Subfertilität aufweisen, was in ursächlichem Zusammenhang mit verminderten Reproduktionsprozessen wie der Ovulation und Fertilisierung zu sehen ist.

Der EP₂-Rezeptor stellt demnach ein wichtiges Ziel für die Entwicklung von Arzneimitteln für die Regulation der weiblichen Fertilität dar. Die Existenz der 4 Subklassen des PGE₂-Rezeptors eröffnet die Möglichkeit zur gezielten Entwicklung selektiv wirksamer Verbindungen. Bisher sind allerdings kaum selektive EP₂-Rezeptor-Liganden bekannt, die an den EP₂-Subtypen des PGE₂-Rezeptors binden, da die meisten bekannten Verbindungen auch an die anderen PGE₂-Rezeptor-Subtypen, wie beispielsweise an den EP₄-Rezeptor, binden.

EP₂-Rezeptor Antagonisten werden beispielsweise in der Anmeldung US2005059742 beschrieben (Jabbour, Medical Research Concil). Beansprucht wird eine Methode, bei der ein EP₂ und/oder ein EP₄ Antagonist zur Behandlung von Menorrhagie und Dysmenorrhoe eingesetzt werden kann. AH6809 wird als Antagonist des EP₂ oder EP₄ Rezeptors offenbart, es werden keine anderen spezifischen Antagonisten und keine neuen Verbindungen offenbart.

In einer früheren Anmeldung derselben Gruppe (EP1467738) werden EP₂ oder EP₄ Antagonisten zur Behandlung von pathologischen Zuständen beschrieben wie z.B. allergische Erkrankungen, Morbus Alzheimer, Schmerz, Abort, Menstruationsbeschwerden, Menorrhagie und Dysmenorrhoe, Endometriose, Erkrankungen der Knochen, Ischämie usw. Die beschriebenen Verbindungen zeichnen sich jedoch durch eine besonders hohe Affinität zum EP₃ Rezeptor aus. In einer weiteren Anmeldung (WO04/032964) werden neue Verbindungen beschrieben, die sich ebenfalls durch eine besonders hohe Affinität zum EP₃ Rezeptor auszeichnen, aber auch EP₂ antagonistisch wirken und die für Behandlung und Prophylaxe von allergischen Erkrankungen Verwendung finden.

Ono Pharmaceutical beansprucht in der Anmeldung WO03/016254 die Herstellung von Benzolsäure oder gesättigten Carbonsäure-Derivaten, die mit Aryl oder Heterocyclen substituiert sind, unter anderem als PGE₂-Rezeptor Antagonisten. Die offenbarten Verbindungen werden für die Behandlung einer Vielzahl von Erkrankungen beansprucht, darunter auch allergische Erkrankungen, Morbus Alzheimer, Schmerz, Abort, Menstruationsbeschwerden, Menorrhagie und Dysmenorrhoe, Endometriose, Erkrankungen der Knochen, Ischämie usw. Die beschriebenen Verbindungen zeichnen sich jedoch durch eine besonders hohe Affinität zum EP₃ Rezeptor aus. In einer weiteren Anmeldung (WO04/032964) werden neue Verbindungen beschrieben, die sich ebenfalls durch eine besonders hohe Affinität zum EP₃ Rezeptor auszeichnen, aber auch als EP₂ Antagonisten, für die Behandlung und Prophylaxe von allergischen Erkrankungen Verwendung finden.

In der Anmeldung WO04/39807 der Firma Merck Frosst, Canada, wird die Herstellung von Pyridopyrrolizinen und Pyridoindolizinen offenbart. Diese Verbindungen zeichnen sich jedoch durch gute Bindung an den PGD₂ Rezeptor aus, dieser Rezeptor stellt einen anderen Subtyp des Prostaglandinrezeptors dar.

Naphathalinderivate als EP₄-Rezeptor Liganden werden von der SmithKline Beecham Cooperation in der Anmeldung US2004102508 offenbart. Die beanspruchten Verbindungen finden ihre Verwendung für die Behandlung oder Prophylaxe von Schmerz, allergischen Reaktionen und neurodegenerativer Erkrankungen.

EP₄-Antagonisten (□-Lactame) werden in der Anmeldung WO03/103604 beansprucht (Applied Research Systems). Die Verbindungen binden ca. 60fach besser an den EP₄- als an den EP₂-Rezeptor und werden unter anderem zur Behandlung von vorzeitigen Wehen, Dysmenorrhoe, Asthma, Unfruchtbarkeit oder Fertilitätsstörungen beansprucht. Von derselben Firma werden in den Anmeldungen WO03/053923 (substituierte Pyrrolidine) oder WO03/035064 (substituierte Pyrazolidione) Verbindungen für die Behandlung von Erkrankungen, die mit Prostaglandinen assoziiert sind, wie beispielsweise Infertilität, Hypertension und Osteoporose, beansprucht. Die Verbindungen binden an den EP₄-und an den EP₂ Rezeptor-Subtypen. In der Anmeldung WO03/037433 werden ω-Cycloalkyl, 17 Heteroaryl-Prostaglandinderivate als EP₂-Rezeptor Antagonisten, insbesondere für die Behandlung von erhöhtem Augeninnendruck, beansprucht.

In der Anmeldung WO03/064391 (Pfizer Products) werden Metabolite von [3-[[N-(4-tert-butylbenzyl)(pyridin-3-ylsulfonyl)amino]methyl] Essigsäure beschrieben, die die Bindung von [³H] Prostaglandin-E₂ an den EP₂-Rezeptor inhibieren. Die Verwendung dieser Metabolite zur Behandlung von Osteoporose wird offenbart.
Tani *et al.* beanspruchen in der Anmeldung US2005124577 8-Azaprostaglandinderivate für die Behandlung von immunologischen Erkrankungen, allergischen Erkrankungen, vorzeitigen Wehen, Abort usw. Die Verbindungen binden an den EP₂ und an den EP₄ Rezeptor.

In der europäischen Patentanmeldung EP 1306087 werden EP₂-Rezeptor Agonisten beschrieben, die ihre Verwendung bei der Behandlung der erektilen Dysfunktion finden (Ono Pharmaceuticals). Die gleiche Strukturklasse wird in dem europäischen Patent EP 860430 (Ono Pharmaceuticals) beschrieben, ihre Verwendung für die Herstellung eines Medikaments für die Behandlung von immunologischen Erkrankungen, Asthma und Abort wird beansprucht. In der WO04/009117 werden EP₂- und EP₄-Rezeptor Agonisten für die Behandlung von Erkrankungen beschrieben, die verursacht werden durch die Uteruskontraktion, beispielsweise Menstruationsbeschwerden (Ono Pharmaceuticals).

In den Anmeldungen WO03/74483 und WO03/09872 werden Agonisten beschrieben, die gleichermaßen an den EP₂ und an den EP₄ Rezeptor binden (Ono Pharmaceuticals).

Die Agonisten des EP₂ und des EP₄ Rezeptors werden häufig im Zusammenhang mit der Behandlung der Osteoporose beschrieben (WO99/19300 (Pfizer), US2003/0166631 (Dumont Francis), WO03/77910 (Pfizer), WO03/4537 (Pfizer), WO03/74483 und WO03/09872 (Ono Pharmaceuticals)) und für die Glaukombehandlung (WO04/37813, WO04/37786, WO04/19938, WO03/103772, WO03/103664, WO03/40123 WO03/47513 WO03/4741 (Merck Frosst Canada)) und US6410591 und US6747037 (Allergan).

In der Patentanmeldung WO04/12656 (Applied Research Systems) werden EP₂-Rezeptor Agonisten im Zusammenhang mit Inflammation beansprucht.
In der Patentanmeldung WO03/77919 (Merck & Co. Inc.) werden EP₄-Rezeptor Agonisten für die Behandlung der Fertilität beansprucht.

In der Patentanmeldung WO95/19774 (Warner-Lambert Company) werden Thienopyrimidine als Inhibitoren der Tyrosinkinase des EGF-Rezeptors beschrieben. Die Verwendung der in der Anmeldung offenbarten Verbindungen für Krebsbehandlung, Pankreatitis und Fertilitätskontrolle wird beansprucht. Allerdings wird die Wirksamkeit der Verbindungen nur in Hinblick auf die Kinaseinhibition gezeigt.

Aus der Anmeldung WO 07/71456 (Bayer Schering Pharma) sind Carbazole und Fluorene bekannt, die selektiv im µM Bereich an den EP₂-Rezeptor binden. Nach wie vor besteht jedoch Bedarf nach wirksameren EP₂-Rezeptor Modulatoren für die Regulation der Prozesse, die letztendlich für die Ovulation, Fertilisierung, Nidation und Dezidualisierung verantwortlich sind und somit zur Förderung oder Hemmung der Fertilität beitragen.

Aufgabe der vorliegenden Erfindung ist es daher, wirksamere EP₂-Rezeptor Modulatoren zur Verfügung zu stellen.

Diese Aufgabe wird durch die Verbindungen der allgemeinen Formel I gelöst, 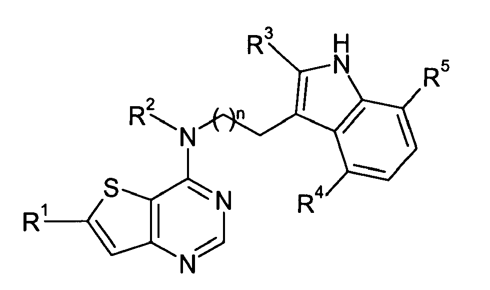 wobei
- R¹: ein Wasserstoff, Halogen, eine C₁-C₄-Alkylgruppe,
eine CH=CH-Arylgruppe mit einem 6-10-gliedrigen, mono- oder bicyclischen Arylring, der jeweils unsubstituiert oder gegebenenfalls ein- bis dreifach substituiert ist,
eine CH=CH-Heteroarylgruppe mit einem 5-10-gliedrigen, mono- oder bicyclischen Heteroarylring, der jeweils unsubstituiert oder gegebenenfalls ein- bis dreifach substituiert ist,
ein 6-10-gliedriger, mono- oder bicyclischer Arylring, der jeweils unsubstituiert oder gegebenenfalls ein- bis dreifach substituiert ist, ein 5-10-gliedriger, mono- oder bicyclischer Heteroarylring, der jeweils unsubstituiert oder gegebenenfalls ein- bis dreifach substituiert ist,
wobei die Substituenten jeweils ausgewählt sein können aus der Gruppe
Halogen, Cyano, CH₂CN, R⁶, OR⁶, CH₂OR⁶, OC(O)R⁶, S(O)ₙR⁶, wobei n = 0, 1, 2 bedeutet,
SO₂NHR⁶, SO₂NHC(O)R⁶, NR⁶R⁷, NHC(O)R⁶, NHSO₂R⁶, CH₂NR⁶R⁷, CH₂NHC(O)R⁶, C(OH)R⁶R⁷, C(O)R⁶, CO₂R⁶, C(O)NR⁶R⁷,
- n: 1 oder 2,
- R²: ein Wasserstoff, eine C₁-C₄-Alkylgruppe,
- R³: ein Wasserstoff, eine C₁-C₄-Alkylgruppe,
- R⁴: ein Wasserstoff, Halogen, Cyano, eine C₁-C₄-Alkylgruppe,
- R⁵: ein Wasserstoff, Halogen, Cyano, eine C₁-C₄-Alkylgruppe,
- R⁶: ein Wasserstoff, eine C₁-C₄-Alkylgruppe, eine C₂-C₄-Alkenylgruppe, eine C₂-C₄-Alkinylgruppe, eine C₃-C₆-Cycloalkylgruppe, eine CH₂-C₃-C₆-Cycloalkylgruppe, ein 6-gliedriger Arylring, ein 5-6-gliedriger Heteroarylring oder eine CH₂-Aryl- bzw. Heteroarylgruppe, wobei der Arylrest 6-gliedrig und der Heteroarylrest 5 bzw. 6-gliedrig ist,
- R⁷: ein Wasserstoff, eine C₁-C₄-Alkylgruppe,
- R⁶, R⁷: zusammen einen 3-6-gliedrigen Ring bilden,

sowie deren Stereoisomere, Diastereomere, Enantiomere, Salze und deren Cyclodextrinclathraten bedeuten und die die bekannten Nachteile überwinden, und eine bessere Selektivität zum EP₂ Rezeptor und somit eine bessere Wirksamkeit und längere Wirkdauer erzielen..

Bei dem unter R¹-R⁷ angegebenen gesättigten, unverzweigten C₁-C₄-Alkylsubstituenten handelt es sich beispielsweise um eine Methyl-, Ethyl-, n-Propyl-, n-Butyl-, bei den verzweigten C₃-C₄-Alkylgruppen um eine iso-Propyl-, *iso*-Butyl-, *sec*-Butyl, *tert*-Butylgruppe. Die Alkylgruppen können gegebenenfalls ein- oder mehrfach substituiert sein mit Halogenatomen (z.B. Fluor, Chlor oder Brom).

Die C₂-C₄-Alkenyl-Substituenten in R⁶ sind jeweils geradkettig oder verzweigt, wobei beispielsweise folgenden Reste gemeint sind: Vinyl-, Allyl-, Homoallyl-, (E)-But-2-enyl-, (Z)-But-2-enyl-, 2-Methylvinyl-.
Die Alkenylgruppen können gegebenenfalls ein- oder mehrfach substituiert sein mit Halogenatomen (z.B. Fluor, Chlor oder Brom).

Die C₂-C₄-Alkinyl-Substituenten in R⁶ sind jeweils geradkettig oder verzweigt,
wobei beispielsweise folgenden Reste gemeint sind: Ethinyl, Prop-1-inyl, But-1-inyl, But-2-inyl.
Die Alkinylgruppen können gegebenenfalls einfach substituiert sein mit Halogenatomen (z.B. Fluor, Chlor oder Brom).

Unter Halogen werden folgende verstanden: Fluor, Chlor, Brom, Iod.

Bei der in R⁶ angegebenen C₃-C₆-Cycloalkylgruppe handelt es sind um Alkylringe wie Cyclopropyl, Cyclobutyl, Cyclopentyl oder Cyclohexyl sowie um Heteroatom-haltige Heterocyclen wie beispielsweise Aziridinyl, Azetidinyl, Pyrrolidinyl, Tetrahydrofuranyl, Piperidinyl, Piperazinyl, Morpholinyl, Thiomorpholinyl.

Die C₃-C₆-Cycloalkylgruppen sind über eine der substituierbaren Stellen verknüpft und können gegebenenfalls ein- bis zweifach substituiert sein mit Halogenatomen (z.B. Fluor, Chlor oder Brom). Die N- bzw. S-Atome können gegebenenfalls oxidiert sein zu einem N-Oxid, S-Oxid, S,S-Dioxid.

Der in R¹ angegebene 6-10-gliedrige, mono- oder bicyclische Arylrest, der gegebenenfalls ein- bis dreifach substituiert sein kann, ist über eine der möglichen Verknüpfungsstellen mit dem Gerüst verbunden.
Beispielsweise seien für einen 6-10-gliedrigen, mono- oder bicyclischen Arylrest folgende genannt: Phenyl, Naphthyl.

Unter dem in R¹ angegebenen 5-10-gliedrigen, mono- oder bicyclischen Heteroarylrest, der gegebenenfalls ein- bis dreifach substituiert sein kann, werden 5-10-gliedrige Ringsysteme verstanden, die anstelle des Kohlenstoffs ein oder mehrere, gleiche oder verschiedene Heteroatome, wie Sauerstoff, Stickstoff oder Schwefel im Ring enthalten können, mono- oder bicyclisch sein können und über eine der möglichen Verknüpfungsstellen mit dem Gerüst verbunden sind. Die 5-10-gliedrigen, mono- oder bicyclischen Heteroarylreste können gegebenenfalls ein- bis dreifach substituiert sein. Ist der Heteroarylrest mit einer Hydroxygruppe substituiert, sind die entsprechenden Tautomere eingeschlossen, sofern die Hydroxygruppe am Heteroarylrest dazu in der Lage ist. Die N-Atome können gegebenenfalls oxidiert sein zu einem N-Oxid.
Bei den 5-10-gliedrigen, mono- oder bicyclischen Heteroarylresten kann es sich um eine über eine der substituierbaren Stellen verknüpfte Pyridyl-, Pyrimidyl-, Chinolinyl-, Isochinolinyl-, Phthalazinyl-, Chinazolinyl-, Chinoxalinyl-, Cinnolinyl-, Benzofuranyl-, Benzothienyl-, Indolyl-, Benzimidazolyl, 2,1,3-Benzothiadiazolyl-, 1H-Benzotriazolyl-, Benzothiazolyl-, Benzoxazolyl-, Benzisoxazolyl-, Furanyl-, Thienyl-, Oxazolyl-, Isoxazolyl-, Isothiazolyl-, Thiazolyl-, Isothiazolyl-, Pyrrolyl-, Pyrazolyl-, Pyrazinyl-, Pyridazinyl-, Triazinyl-, 1H-Pyrazolo[3,4-d]pyrimidyl-, 1H-Indazolyl-, Triazolyl-, Oxadiazolyl-, Tetrazolyl- oder eine Imidazolyl-Gruppe handeln.

Bei dem in R⁶ angegebenen 6-gliedrigen Arylrest handelt es sich um einen Phenylrest, der gegebenenfalls ein- bis zweifach substituiert sein kann mit Halogenatomen (z.B. Fluor, Chlor oder Brom), C₁-C₄-Alkylgruppen, einer Cyanogruppe oder einer Hydroxygruppe.

Unter dem in R⁶ angegebenen 5-6-gliedrigen Heteroarylrest werden 5-6-gliedrige Ringsysteme verstanden, die anstelle des Kohlenstoffs ein oder mehrere, gleiche oder verschiedene Heteroatome, wie Sauerstoff, Stickstoff oder Schwefel im Ring enthalten können, und über eine der möglichen Verknüpfungsstellen mit dem Gerüst verbunden sind. Die 5-6-gliedrigen Heteroarylreste können gegebenenfalls ein- bis zweifach substituiert sein mit Halogenatomen (z.B. Fluor, Chlor oder Brom), C₁-C₄-Alkylgruppen, einer Cyanogruppe oder einer Hydroxygruppe. Ist der Heteroarylrest mit einer Hydroxygruppe substituiert, sind die entsprechenden Tautomere eingeschlossen, sofern die Hydroxygruppe am Heteroarylrest dazu in der Lage ist. Die N-Atome können gegebenenfalls oxidiert sein zu einem N-Oxid.
Bei den 5-6-gliedrigen Heteroarylgruppen kann es sich um eine über eine der substituierbaren Stellen verknüpfte Pyridyl-, Pyrimidyl-, Furanyl-, Thienyl-, Oxazolyl-, Isoxazolyl-, Thiazolyl-, Isothiazolyl-, Pyrrolyl-, Pyrazolyl-, Pyrazinyl-, Pyridazinyl-, Triazinyl-, Triazolyl-, Oxadiazolyl-, Tetrazolyl- oder eine Imidazolyl-Gruppe handeln.

Bei dem 3-6-gliedrigen Ring, der durch Ringschluss von R⁶ und R⁷ gebildet werden kann, kann es sich um einen Cycloalkylring oder einen Heteroatom-haltigen Cyclus handeln. Als Beispiele für einen 3-6-gliedrigen Cycloalkylring seinen beispielsweise folgende genannt: Cyclopropyl, Cyclobutyl, Cyclopentyl oder Cyclohexyl.

Als Beispiel für einen 3-6-gliedrigen, Heteroatom-haltigen Heterocyclus seinen beispielsweise folgende genannt: Aziridinyl, Azetidinyl, Pyrrolidinyl, Piperidinyl, Piperazinyl, Morpholinyl, Thiomorpholinyl. Die N- bzw. S-Atome können gegebenenfalls oxidiert sein zu einem N-Oxid, S-Oxid, S,S-Dioxid.

Bevorzugt sind die Verbindungen der allgemeinen Formel 1, wobei
- R¹: ein Wasserstoff, Halogen, eine C₁-C₄-Alkylgruppe,
eine CH=CH-Arylgruppe mit einem 6-10-gliedrigen, mono- oder bicyclischen Arylring, der jeweils unsubstituiert oder gegebenenfalls ein- bis dreifach substituiert ist,
eine CH=CH-Heteroarylgruppe mit einem 5-10-gliedrigen, mono- oder bicyclischen Heteroarylring, der jeweils unsubstituiert oder gegebenenfalls ein- bis dreifach substituiert ist,
ein 6-10-gliedriger, mono- oder bicyclischer Arylring, der jeweils unsubstituiert oder gegebenenfalls ein- bis dreifach substituiert ist, ein 5-10-gliedriger, mono- oder bicyclischer Heteroarylring, der jeweils unsubstituiert oder gegebenenfalls ein- bis dreifach substituiert ist,
wobei die Substituenten jeweils ausgewählt sein können aus der Gruppe
Halogen, Cyano, CH₂CN, R⁶, OR⁶, CH₂OR⁶, OC(O)R⁶, S(O)ₙR⁶, wobei n = 0, 1, 2 bedeutet, SO₂NHR⁶, SO₂NHC(O)R⁶, NR⁶R⁷, NHC(O)R⁶, NHSO₂R⁶, CH₂NR⁶R⁷, CH₂NHC(O)R⁶, C(OH)R⁶R⁷, C(O)R⁶, CO₂R⁶, C(O)NR⁶R⁷,
- n: 1 oder 2,
- R²: ein Wasserstoff, eine Methylgruppe,
- R³: ein Wasserstoff, eine C₁-Alkylgruppe,
- R⁴: ein Wasserstoff, Halogen, Cyano, eine C₁-C₄-Alkylgruppe,
- R⁵: ein Wasserstoff, Halogen, Cyano, eine C₁-C₄-Alkylgruppe,
- R⁶: ein Wasserstoff, eine C₁-C₄-Alkylgruppe, eine C₂-C₄-Alkenylgruppe, eine C₂-C₄-Alkinylgruppe, eine C₃-C₆-Cycloalkylgruppe, eine CH₂- C₃-C₆-Cycloalkylgruppe, ein 6-gliedriger Arylring, ein 5-6-gliedriger Heteroarylring oder eine CH₂-Aryl- bzw. Heteroarylgruppe, wobei der Arylrest 6-gliedrig und der Heteroarylrest 5 bzw. 6-gliedrig ist,
- R⁷: ein Wasserstoff, eine C₁-C₄-Alkylgruppe,
- R⁶, R⁷: zusammen einen 3-6-gliedrigen Ring bilden,
bedeuten.

Ebenfalls bevorzugt sind die Verbindungen der allgemeinen Formel 1, wobei
- R¹: ein Wasserstoff, Halogen, eine CH=CH-Arylgruppe mit einem 6-10-gliedrigen, mono- oder bicyclischen Arylring, der jeweils unsubstituiert oder gegebenenfalls ein- bis dreifach substituiert ist, eine CH=CH-Heteroarylgruppe mit einem 5-10-gliedrigen, mono- oder bicyclischen Heteroarylring, der jeweils unsubstituiert oder gegebenenfalls ein- bis dreifach substituiert ist,
ein 6-10-gliedriger, mono- oder bicyclischer Arylring, der jeweils unsubstituiert oder gegebenenfalls ein- bis dreifach substituiert ist, ein 5-10-gliedriger, mono- oder bicyclischer Heteroarylring, der jeweils unsubstituiert oder gegebenenfalls ein- bis dreifach substituiert ist,
wobei die Substituenten jeweils ausgewählt sein können aus der Gruppe
Halogen, Cyano, CH₂CN, R⁶, OR⁶, CH₂OR⁶, OC(O)R⁶, S(O)ₙR⁶, wobei n = 0, 1, 2 bedeutet,
SO₂NHR⁶, SO₂NHC(O)R⁶, NR⁶R⁷, NHC(O)R⁶, NHSO₂R⁶, CH₂NR⁶R⁷, CH₂NHC(O)R⁶, C(OH)R⁶R⁷, C(O)R⁶, CO₂R⁶, C(O)NR⁶R⁷,
- n: 1 oder 2,
- R²: ein Wasserstoff, eine Methylgruppe,
- R³: ein Wasserstoff, eine C₁-Alkylgruppe,
- R⁴: ein Wasserstoff, Halogen, Cyano, eine C₁-C₄-Alkylgruppe,
- R⁵: ein Wasserstoff, Halogen, Cyano, eine C₁-C₄-Alkylgruppe,
- R⁶: ein Wasserstoff, eine C₁-C₄-Alkylgruppe, eine C₂-C₄-Alkenylgruppe, eine C₂-C₄-Alkinylgruppe, eine C₃-C₆-Cycloalkylgruppe, eine CH₂-C₃-C₆-Cycloalkylgruppe, ein 6-gliedriger Arylring, ein 5-6-gliedriger Heteroarylring oder eine CH₂-Aryl- bzw. Heteroarylgruppe, wobei der Arylrest 6-gliedrig und der Heteroarylrest 5 bzw. 6-gliedrig ist,
- R⁷: ein Wasserstoff, eine C₁-C₄-Alkylgruppe,
- R⁶, R⁷: zusammen einen 3-6-gliedrigen Ring bilden, bedeuten.

Besonders bevorzugt sind die Verbindungen der allgemeinen Formel 1, wobei
- R¹: ein Wasserstoff, Halogen,
eine CH=CH-Phenylgruppe, wobei der Phenylring jeweils unsubstituiert oder gegebenenfalls ein- bis dreifach substituiert ist,
eine CH=CH-Heteroarylgruppe mit einem 5-6-gliedrigen Heteroarylring, der jeweils unsubstituiert oder gegebenenfalls ein- bis dreifach substituiert ist,
ein 6-10-gliedriger, mono- oder bicyclischer Arylring, der jeweils unsubstituiert oder gegebenenfalls ein- bis dreifach substituiert ist, ein 5-10-gliedriger, mono- oder bicyclischer Heteroarylring, der jeweils unsubstituiert oder gegebenenfalls ein- bis dreifach substituiert ist,
wobei die Substituenten jeweils ausgewählt sein können aus der Gruppe
Halogen, Cyano, CH₂CN, R⁶, OR⁶, CH₂OR⁶, OC(O)R⁶, S(O)ₙR⁶, wobei n = 0, 1, 2 bedeutet,
SO₂NHR⁶, SO₂NHC(O)R⁶, NR⁶R⁷, NHC(O)R⁶, NHSO₂R⁶, CH₂NR⁶R⁷, CH₂NHC(O)R⁶, C(OH)R⁶R⁷, C(O)R⁶, CO₂R⁶, C(O)NR⁶R⁷,
- n: 1 oder 2,
- R²: ein Wasserstoff, eine Methylgruppe,
- R³: ein Wasserstoff, eine C₁-Alkylgruppe,
- R⁴: ein Wasserstoff, Halogen, Cyano, eine C₁-C₄-Alkylgruppe,
- R⁵: ein Wasserstoff, Halogen, Cyano, eine C₁-C₄-Alkylgruppe,
- R⁶: ein Wasserstoff, eine C₁-C₄-Alkylgruppe, eine C₂-C₄-Alkenylgruppe, eine C₂-C₄-Alkinylgruppe, eine C₃-C₆-Cycloalkylgruppe, eine CH₂-C₃-C₆-Cycloalkylgruppe, ein 6-gliedriger Arylring, ein 5-6-gliedriger Heteroarylring oder eine CH₂-Aryl- bzw. Heteroarylgruppe, wobei der Arylrest 6-gliedrig und der Heteroarylrest 5 bzw. 6-gliedrig ist,
- R⁷: ein Wasserstoff, eine C₁-C₄-Alkylgruppe,
- R⁶, R⁷: zusammen einen 3-6-gliedrigen Ring bilden,
bedeuten.

Die folgenden Verbindungen entsprechend der vorliegenden Erfindung sind ganz besonders bevorzugt:
1. [2-(7-Fluor-2,4-dimethyl-1H-indol-3-yl)-ethyl]-(6-phenyl-thieno[3,2-d]pyrimidin-4-yl)-amin
2. [2-(7-Fluor-2,4-dimethyl-1H-indol-3-yl)-ethyl]-(6-o-tolyl-thieno[3,2-d]pyrimidin-4-yl)-amin
3. [2-(7-Fluor-2,4-dimethyl-1H-indol-3-yl)-ethyl]-(6-thiophen-3-yl-thieno[3,2-d]pyrimidin-4-yl)-amin
4. 2-(7-Fluor-2,4-dimethyl-1H-indol-3-yl)-ethyl]-(6-thiophen-2-yl-thieno[3,2-d]pyrimidin-4-yl)-amin
5. [2-(7-Fluor-2,4-dimethyl-1H-indol-3-yl)-ethyl]-[6-(4-methoxy-phenyl)-thieno[3,2-d]pyrimidin-4-yl]-amin
6. 2-(7-Fluor-2,4-dimethyl-1H-indol-3-yl)-ethyl]-(6-naphthalen-2-yl-thieno[3,2-d]pyrimidin-4-yl)-amin
7. (6-Biphenyl-4-yl-thieno[3,2-d]pyrimidin-4-yl)-[2-(7-fluor-2,4-dimethyl-1H-indol-3-yl)-ethyl]-amin
8. (6-Benzofuran-2-yl-thieno[3,2-d]pyrimidin-4-yl)-[2-(7-fluor-2,4-dimethyl-1H-indol-3-yl)-ethyl]-amin
9. [2-(7-Fluor-2,4-dimethyl-1H-indol-3-yl)-ethyl]-(6-m-tolyl-thieno[3,2-d]pyrimidin-4-yl)-amin
10. [2-(7-Fluor-2,4-dimethyl-1H-indol-3-yl)-ethyl]-(6-p-tolyl-thieno[3,2-d]pyrimidin-4-yl)-amin
11. (6-Benzo[b]thiophen-2-yl-thieno[3,2-d]pyrimidin-4-yl)-[2-(7-fluor-2,4-dimethyl-1H-indol-3-yl)-ethyl]-amin
12. [2-(7-Fluor-2,4-dimethyl-1H-indol-3-yl)-ethyl]-(6-naphthalen-1-yl-thieno[3,2-d]pyrimidin-4-yl)-amin
13. [2-(7-Fluor-2,4-dimethyl-1H-indol-3-yl)-ethyl]-[6-(3-fluor-4-methyl-phenyl)-thieno[3,2-d]pyrimidin-4-yl]-amin
14. (4-{4-[2-(7-Fluor-2,4-dimethyl-1H-indol-3-yl)-ethylamino]-thieno[3,2-d]pyrimidin-6-yl}-phenyl)-methanol
15. 4-{4-[2-(7-Fluor-2,4-dimethyl-1H-indol-3-yl)-ethylamino]-thieno[3,2-d]pyrimidin-6-yl}-benzonitril
16. [2-(7-Fluor-2,4-dimethyl-1H-indol-3-yl)-ethyl]-[6-(4-fluor-phenyl)-thieno[3,2-d]pyrimidin-4-yl]-amin
17. [2-(7-Fluor-2,4-dimethyl-1H-indol-3-yl)-ethyl]-[6-(5-fluor-2-methoxyphenyl)-thieno[3,2-d]pyrimidin-4-yl]-amin
18. 1-(4-{4-[2-(7-Fluor-2,4-dimethyl-1H-indol-3-yl)-ethylamino]-thieno[3,2-d]pyrimidin-6-yl}-phenyl)-ethanon
19. [6-(4-tert-Butyl-phenyl)-thieno[3,2-d]pyrimidin-4-yl]-[2-(7-fluor-2,4-dimethyl-1H-indol-3-yl)-ethyl]-amin
20. [6-(3,5-Bis-trifluormethyl-phenyl)-thieno[3,2-d]pyrimidin-4-yl]-[2-(7-fluor-2,4-dimethyl-1H-indol-3-yl)-ethyl]-amin
21. [6-(4-Chlor-phenyl)-thieno[3,2-d]pyrimidin-4-yl]-[2-(7-fluor-2,4-dimethyl-1H-indol-3-yl)-ethyl]-amin
22. [2-(7-Fluor-2,4-dimethyl-1H-indol-3-yl)-ethyl]-[6-(3-fluor-phenyl)-thieno[3,2-d]pyrimidin-4-yl]-amin
23. [2-(7-Fluor-2,4-dimethyl-1H-indol-3-yl)-ethyl]-[6-(2-trifluormethoxyphenyl)-thieno[3,2-d]pyrimidin-4-yl]-amin
24. (6-Biphenyl-3-yl-thieno[3,2-d]pyrimidin-4-yl)-[2-(7-fluor-2,4-dimethyl-1H-indol-3-yl)-ethyl]-amin
25. [2-(7-Fluor-2,4-dimethyl-1H-indol-3-yl)-ethyl]-[6-(2-phenoxy-phenyl)-thieno[3,2-d]pyrimidin-4-yl]-amin
26. (3-{4-[2-(7-Fluor-2,4-dimethyl-1H-indol-3-yl)-ethylamino]-thieno[3,2-d]pyrimidin-6-yl}-phenyl)-methanol
27. [2-(7-Fluor-2,4-dimethyl-1H-indol-3-yl)-ethyl]-[6-(3-methylsulfanyl-phenyl)-thieno[3,2-d]pyrimidin-4-yl]-amin
28. [6-(2-Chlor-phenyl)-thieno[3,2-d]pyrimidin-4-yl]-[2-(7-fluor-2,4-dimethyl-1H-indol-3-yl)-ethyl]-amin
29. (6-Benzo[b]thiophen-3-yl-thieno[3,2-d]pyrimidin-4-yl)-[2-(7-fluor-2,4-dimethyl-1H-indol-3-yl)-ethyl]-amin
30. [2-(7-Fluor-2,4-dimethyl-1H-indol-3-yl)-ethyl]-[6-(4-trifluormethyl-phenyl)-thieno[3,2-d]pyrimidin-4-yl]-amin
31. [2-(7-Fluor-2,4-dimethyl-1H-indol-3-yl)-ethyl]-[6-((E)-styryl)-thieno[3,2-d]pyrimidin-4-yl]-amin
32. [2-(7-Fluor-2,4-dimethyl-1H-indol-3-yl)-ethyl]-(6-chinolin-6-yl-thieno[3,2-d]pyrimidin-4-yl)-amin
33. [2-(7-Fluor-2,4-dimethyl-1H-indol-3-yl)-ethyl]-[6-(3-pyrrolidin-1-yl-phenyl)-thieno[3,2-d]pyrimidin-4-yl]-amin
34. 2-Fluor-5-{4-[2-(7-fluor-2,4-dimethyl-1H-indol-3-yl)-ethylamino]-thieno[3,2-d]pyrimidin-6-yl}-benzonitril
35. [2-(7-Fluor-2,4-dimethyl-1H-indol-3-yl)-ethyl]-[6-(6-fluor-5-methyl-pyridin-3-yl)-thieno[3,2-d]pyrimidin-4-yl]-amin
36. [6-(2-Chlor-6-methyl-pyridin-3-yl)-thieno[3,2-d]pyrimidin-4-yl]-[2-(7-fluor-2,4-dimethyl-1H-indol-3-yl)-ethyl]-amin
37. [2-(7-Fluor-2,4-dimethyl-1H-indol-3-yl)-ethyl]-[6-(6-methoxy-pyridin-3-yl)-thieno[3,2-d]pyrimidin-4-yl]-amin
38. [6-(3-Chlor-4-methyl-phenyl)-thieno[3,2-d]pyrimidin-4-yl]-[2-(7-fluor-2,4-dimethyl-1H-indol-3-yl)-ethyl]-amin
39. 4-{4-[2-(7-Fluor-2,4-dimethyl-1H-indol-3-yl)-ethylamino]-thieno[3,2-d]pyrimidin-6-yl)-N-methyl-benzamid
40. (3-{4-[2-(7-Fluor-2,4-dimethyl-1H-indol-3-yl)-ethylamino]-thieno[3,2-d]pyrimidin-6-yl}-phenyl)-acetonitril
41. N-(2-{4-[2-(7-Fluor-2,4-dimethyl-1H-indol-3-yl)-ethylamino]-thieno[3,2-d]pyrimidin-6-yl)-phenyl)-acetamid
42. [2-(7-Fluor-2,4-dimethyl-1H-indol-3-yl)-ethyl]-[6-(5-methyl-furan-2-yl)-thieno[3,2-d]pyrimidin-4-yl]-amin
43. [2-(7-Fluor-2,4-dimethyl-1H-indol-3-yl)-ethyl]-[6-(2-fluor-phenyl)-thieno[3,2-d]pyrimidin-4-yl]-amin
44. [2-(7-Fluor-2,4-dimethyl-1H-indol-3-yl)-ethyl]-[6-(3-fluor-4-methoxyphenyl)-thieno[3,2-d]pyrimidin-4-yl]-amin
45. [6-(3-Chlor-4-fluor-phenyl)-thieno[3,2-d]pyrimidin-4-yl]-[2-(7-fluor-2,4-dimethyl-1H-indol-3-yl)-ethyl]-amin
46. N-(3-{4-[2-(7-Fluor-2,4-dimethyl-1H-indol-3-yl)-ethylamino]-thieno[3,2-d]pyrimidin-6-yl}-phenyl)-methansulfonamid
47. N-(3-{4-[2-(7-Fluor-2,4-dimethyl-1H-indol-3-yl)-ethylamino]-thieno[3,2-d]pyrimidin-6-yl)-phenyl)-acetamid
48. 6-(5-Chlor-thiophen-2-yl)-thieno[3,2-d]pyrimidin-4-yl]-[2-(7-fluor-2,4-dimethyl-1H-indol-3-yl)-ethyl]-amin
49. [6-(3,4-Difluor-phenyl)-thieno[3,2-d]pyrimidin-4-yl]-[2-(7-fluor-2,4-dimethyl-1H-indol-3-yl)-ethyl]-amin
50. [2-(7-Fluor-2,4-dimethyl-1H-indol-3-yl)-ethyl]-[6-(4-methoxy-3,5-dimethylphenyl)-thieno[3,2-d]pyrimidin-4-yl]-amin
51. [6-(3,5-Difluor-phenyl)-thieno[3,2-d]pyrimidin-4-yl]-[2-(7-fluor-2,4-dimethyl-1H-indol-3-yl)-ethyl]-amin
52. [2-(7-Fluor-2,4-dimethyl-1H-indol-3-yl)-ethyl]-[6-(4-methyl-thiophen-2-yl)-thieno[3,2-d]pyrimidin-4-yl]-amin
53. (4-{4-[2-(7-Fluor-2,4-dimethyl-1H-indol-3-yl)-ethylamino]-thieno[3,2-d]pyrimidin-6-yl}-phenyl)-acetonitril
54. [6-(2,3-Difluor-phenyl)-thieno[3,2-d]pyrimidin-4-yl]-[2-(7-fluor-2,4-dimethyl-1H-indol-3-yl)-ethyl]-amin
55. [6-(2,5-Difluor-phenyl)-thieno[3,2-d]pyrimidin-4-yl]-[2-(7-fluor-2,4-dimethyl-1H-indol-3-yl)-ethyl]-amin
56. [6-(4-Benzyloxy-2-fluor-phenyl)-thieno[3,2-d]pyrimidin-4-yl]-[2-(7-fluor-2,4-dimethyl-1H-indol-3-yl)-ethyl]-amin
57. [2-(7-Fluor-2,4-dimethyl-1H-indol-3-yl)-ethyl]-{6-[(E)-2-(4-fluor-phenyl)-vinyl]-thieno[3,2-d]pyrimidin-4-yl}-amin
58. 5-{4-[2-(7-Fluor-2,4-dimethyl-1H-indol-3-yl)-ethylamino]-thieno[3,2-d]pyrimidin-6-yl}-thiophen-2-carbonitril
59.[2-(7-Fluor-2,4-dimethyl-1H-indol-3-yl)-ethyl]-[6-(2-fluor-3-methoxyphenyl)-thieno[3,2-d]pyrimidin-4-yl]-amin
60. [2-(7-Fluor-2,4-dimethyl-1H-indol-3-yl)-ethyl]-[6-(2-methoxy-pyrimidin-5-yl)-thieno[3,2-d]pyrimidin-4-yl]-amin
61. (5-{4-[2-(7-Fluor-2,4-dimethyl-1H-indol-3-yl)-ethylamino]-thieno[3,2-d]pyrimidin-6-yl}-thiophen-2-yl)-methanol
62. [6-(3-Cyclopropylmethoxy-phenyl)-thieno[3,2-d]pyrimidin-4-yl]-[2-(7-fluor-2,4-dimethyl-1H-indol-3-yl)-ethyl]-amin
63. [6-(3,5-Dimethyl-phenyl)-thieno[3,2-d]pyrimidin-4-yl]-[2-(7-fluor-2,4-dimethyl-1H-indol-3-yl)-ethyl]-amin
64. [2-(1H-Indol-3-yl)-ethyl]-[6-(3-methoxy-phenyl)-thieno[3,2-d]pyrimidin-4-yl]-amin
65. [2-(1H-Indol-3-yl)-ethyl]-(6-m-tolyl-thieno[3,2-d]pyrimidin-4-yl)-amin
66. 4-{4-[2-(1H-Indol-3-yl)-ethylamino]-thieno[3,2-d]pyrimidin-6-yl}-benzonitril
67. [6-(4-Fluor-phenyl)-thieno[3,2-d]pyrimidin-4-yl]-[2-(1H-indol-3-yl)-ethyl]-amin
68. (3-{4-[2-(1H-Indol-3-yl)-ethylamino]-thieno[3,2-d]pyrimidin-6-yl}-phenyl)-methanol
69. 4-{4-[2-(1H-Indol-3-yl)-ethylamino]-thieno[3,2-d]pyrimidin-6-yl}-N-methyl-benzamid
70. N-(3-{4-[2-(1H-Indol-3-yl)-ethylamino]-thieno[3,2-d]pyrimidin-6-yl}-phenyl)-methansulfonamid
71. N-(3-{4-[2-(1H-Indol-3-yl)-ethylamino]-thieno[3,2-d]pyrimidin-6-yl}-phenyl)-acetamid
72. 3-{4-[2-(1H-Indol-3-yl)-ethylamino]-thieno[3,2-d]pyrimidin-6-yl}-benzoesäure
73. [3-(1H-Indol-3-yl)-propyl]-[6-(3-methoxy-phenyl)-thieno[3,2-d]pyrimidin-4-yl]-amin
74. [3-(1H-Indol-3-yl)-propyl]-(6-m-tolyl-thieno[3,2-d]pyrimidin-4-yl)-amin
75. [6-(4-Fluor-phenyl)-thieno[3,2-d]pyrimidin-4-yl]-[3-(1H-indol-3-yl)-propyl]-amin
76. (3-{4-[3-(1H-Indol-3-yl)-propylamino]-thieno[3,2-d]pyrimidin-6-yl}-phenyl)-methanol
77. N-(3-{4-[3-(1H-Indol-3-yl)-propylamino]-thieno[3,2-d]pyrimidin-6-yl}-phenyl)-methansulfonamid
78. N-(3-{4-[3-(1H-Indol-3-yl)-propylamino]-thieno[3,2-d]pyrimidin-6-yl}-phenyl)-acetamid
79. 3-{4-[3-(1H-Indol-3-yl)-propylamino]-thieno[3,2-d]pyrimidin-6-yl}-benzoesäure
80. [2-(2,7-Dimethyl-1H-indol-3-yl)-ethyl]-(6-m-tolyl-thieno[3,2-d]pyrimidin-4-yl)-amin
81. 4-{4-[2-(2,7-Dimethyl-1H-indol-3-yl)-ethylamino]-thieno[3,2-d]pyrimidin-6-yl}-benzonitril
82. [2-(2,7-Dimethyl-1H-indol-3-yl)-ethyl]-[6-(4-fluor-phenyl)-thieno[3,2-d]pyrimidin-4-yl]-amin
83. (3-{4-[2-(2,7-Dimethyl-1H-indol-3-yl)-ethylamino]-thieno[3,2-d]pyrimidin-6-yl}-phenyl)-methanol
84. 4-{4-[2-(2,7-Dimethyl-1H-indol-3-yl)-ethylamino]-thieno[3,2-d]pyrimidin-6-yl}-N-methyl-benzamid
85. N-(3-{4-[2-(2,7-Dimethyl-1H-indol-3-yl)-ethylamino]-thieno[3,2-d]pyrimidin-6-yl}-phenyl)-methansulfonamid
86. N-(3-{4-[2-(2,7-Dimethyl-1H-indol-3-yl)-ethylamino]-thieno[3,2-d]pyrimidin-6-yl}-phenyl)-acetamid
87. [2-(7-Fluor-2,4-dimethyl-1H-indol-3-yl)-ethyl]-thieno[3,2-d]pyrimidin-4-yl-amin
88. (6-Brom-thieno[3,2-d]pyrimidin-4-yl)-[2-(7-fluor-2,4-dimethyl-1H-indol-3-yl)-ethyl]-amin
89. [2-(2,7-Dimethyl-1H-indol-3-yl)-ethyl]-(6-phenyl-thieno[3,2-d]pyrimidin-4-yl)-amin
90. [2-(4-Chlor-2,7-dimethyl-1H-indol-3-yl)-ethyl]-(6-phenyl-thieno[3,2-d]pyrimidin-4-yl)-amin
91. (6-Phenyl-thieno[3,2-d]pyrimidin-4-yl)-[2-(2,4,7-trimethyl-1H-indol-3-yl)-ethyl]-amin

Gegenstand der vorliegenden Erfindung sind Arzneimitteln, die mindestens eine der Verbindungen gemäß Formel I enthalten.

Ebenfalls Gegenstand der vorliegenden Erfindung sind Arzneimittel, die die erfindungsgemäßen Verbindungen enthalten mit geeigneten Formulierungs- und Trägerstoffen.

Im Vergleich zu bekannten Prostaglandin E₂-Liganden zeichnen sich die neuen EP₂-Agonisten und Antagonisten durch größere Selektivität und Stabilität aus.

Gegenstand der vorliegenden Erfindung sind Arzneimittel zur Behandlung und Prophylaxe von Erkrankungen, zu denen Fertilitätsstörungen, infektiöse Erkrankungen, Krebs, virale Infektionen, Herz-Kreislauf-Erkrankungen, erhöhter Augeninnendruck, Glaukoma, Erkrankungen des Knochenapparates, angiogenetische Erkrankungen, Störungen der Uteruskontraktion, Schmerz, neuroinflammatorische Erkrankungen, immunomodulatorische Infektionen und nephrologische Erkrankungen zählen.

Unter Fertilitätsstörungen sind die Erkrankungen zu verstehen, die dazu führen, dass keine Ovulation erfolgt, keine Fertilisierung stattfindet, dass die Blastozystenentwicklung beeinträchtigt ist, dass es nicht zur Nidation einer befruchteten Eizelle kommt und keine Dezidualisierung stattfindet, unter infektiösen Erkrankungen sind durch unizelluläre Parasiten hervorgerufene Erkrankungen, unter Krebs solide Tumoren und Leukämie, unter viralen Infektionen z. B. Cytomegalus-Infektionen, Hepatitis, Hepatitis B und C und HIV Erkrankungen, unter immunmodulatorischen Infektionen z.B. Vogelgrippe, unter Herz-Kreislauf-Erkrankungen ischämische Reperfusionserkrankung, Stenosen, Arteriosklerosen und Restenosen, unter angiogenetischen Erkrankungen z.B. Endometriose und Fibrose, unter erhöhtem Augeninnendruck Glaukom, unter Störungen der Uteruskontraktion z.B.

Menstruationsbeschwerden, unter Erkrankungen des Knochenapparates Osteoporose, unter neuroinflammatorischen Erkrankungen Multiple Sklerose, Morbus Alzheimer, Schmerz und unter nephrologischen Erkrankungen Glomerulonephritis, zu verstehen.

Ebenfalls Gegenstand der vorliegenden Erfindung sind Arzneimittel zur Behandlung und Prophylaxe der oben aufgeführten Erkrankungen, die mindestens eine Verbindung gemäß der allgemeinen Formel I enthalten, sowie Arzneimittel mit geeigneten Formulierungs- und Trägerstoffen.

Zur Verwendung der erfindungsgemäßen Verbindungen als Arzneimittel werden diese in die Form eines pharmazeutischen Präparats gebracht, das neben dem Wirkstoff für die enterale oder parenterale Applikation geeignete pharmazeutische, organische oder anorganische inerte Trägermaterialien, wie zum Beispiel, Wasser, Gelatine, Gummi arabicum, Milchzucker, Stärke, Magnesiumstearat, Talk, pflanzliche Öle, Polyalkylenglykole usw. enthält. Die pharmazeutischen Präparate können in fester Form, zum Beispiel als Tabletten, Dragees, Suppositorien, Kapseln, in halbfester Form, zum Beispiel als Salben, Cremes, Gele, Suppositiorien, Emulsionen oder in flüssiger Form, zum Beispiel als Lösungen, Suspensionen oder Emulsionen vorliegen.

Gegebenenfalls enthalten sie Hilfsstoffe, die beispielsweise als Füllstoffe, Bindemeittel, Sprengmittel, Gleitmittel, Lösungsmittel, Lösungsvermittler, Geschmackskorrigentien, Farbstoff, Emulgatoren, fungieren sollen. Hilfsstoffarten im Sinne der Erfindung sind beispielsweise Saccharide (Mono-, Di-, Tri-, Oligo-, und/oder Polysaccharide), Fette, Wachse, Öle, Kohlenwasserstoffe, anionische, nichtionische, kationische natürliche, synthetische oder halbsynthestische Tenside. Gegebenenfalls enthalten sie darüber hinaus Hilfsstoffe, wie Konservierungs-, Stabilisierungs-, Netzmittel oder Emulgatoren; Salze zur Veränderung des osmotischen Drucks oder Puffer. Diese pharmazeutischen Präparate sind ebenfalls Gegenstand der vorliegenden Erfindung.

Zur Inhalation werden zweckmäßigerweise Ärosollösungen hergestellt.

Für die orale Anwendung sind insbesondere Tabletten, Dragees oder Kapseln mit Talkum und/oder Kohlenwasserstoffträger oder -binder, wie zum Beispiel Lactose, Mais- oder Kartoffelstärke, geeignet. Die Anwendung kann auch in flüssiger Form erfolgen, wie zum Beispiel als Saft, dem gegebenenfalls ein Süßstoff beigefügt ist. Für die orale Anwendung derartiger Verbindungen sind ebenfalls auch Clathrate geeignet, beispielsweise seien genannt die Clathrate mit alpha-, beta-, gamma-Cyclodextrin oder auch beta-hydroxypropyl-Cyclodextrin.

Für die parenterale Verabreichung werden sterile, injizierbare, wässrige oder ölige Lösungen benutzt. Besonders geeignet sind Injektionslösungen oder Suspensionen, insbesondere wässrige Lösungen der aktiven Verbindungen in polyethoxyliertem Rizinusöl.

Für die vaginale Applikation sind z. B. Zäpfchen, Tampons oder intratauriner Device geeignet und üblich.

Für die intraartikuläre Injektion können entsprechend zubereitete Kristallsuspensionen verwendet werden.

Für die intramuskuläre Injektion können wässrige und ölige Injektionslösungen oder Suspensionen und entsprechende Depotpräparationen Verwendung finden.

Für die rektale Applikation können die neuen Verbindungen in Form von Suppositorien, Kapseln, Lösungen (z.B. in Form von Klysmen) und Salben sowohl zur systemischen, als auch zur lokalen Therapie verwendet werden.

Zur pulmonalen Applikation der neuen Verbindungen können diese in Form von Ärosolen und Inhalaten verwendet werden.

Für die lokale Anwendung an Augen, äußerem Gehörgang, Mittelohr, Nasenhöhle und Nasennebenhöhlen können die neuen Verbindungen als Tropfen, Salben und Tinkturen in entsprechenden pharmazeutischen Zubereitungen verwendet werden.

Für die topische Auftragung sind Formulierungen in Gelen, Salben, Fettsalben, Cremes, Pasten, Puder, Milch und Tinkturen möglich. Die Dosierung der Verbindungen der allgemeinen Formel I sollte in diesen Zubereitungen 0.01 % - 20 % betragen, um eine ausreichende pharmakologische Wirkung zu erzielen.

Die Dosierung der Wirkstoffe kann je nach Verabfolgungsweg, Alter und Gewicht des Patienten, Art und Schwere der zu behandelnden Erkrankung und ähnlichen Faktoren variieren. Die Behandlung kann durch Einzeldosierungen oder durch eine Vielzahl von Dosierungen über einen längeren Zeitraum erfolgen. Die tägliche Dosis beträgt 0,5 - 1000 mg, vorzugsweise 50 - 200 mg, wobei die Dosis als einmal zu verabreichende Einzeldosis oder unterteilt in 2 oder mehrere Tagesdosen gegeben werden kann.

Als Trägersysteme können auch grenzflächenaktive Hilfsstoffe wie Salze der Gallensäuren oder tierische oder pflanzliche Phospholipide, aber auch Mischungen davon sowie Liposome oder deren Bestandteile verwendet werden.

Die oben beschrieben Formulierungen und Darreichungsformen sind ebenfalls Gegenstand der vorliegenden Erfindung.

Die Administration der erfindungsgemäßen Verbindungen kann durch jede konventionelle Methode erfolgen, einschließlich oraler und parenteraler, z.B. durch subcutane oder intramuskuläre Injektionen. Die enteralen, parenteralen, vaginalen und oralen Applikationen sind ebenfalls Gegenstand der vorliegenden Erfindung.

Die erfindungsgemäßen Verbindungen der allgemeinen Formel I binden an den EP₂ Rezeptor und haben agonistische oder antagonistische Wirkung. Es kann durch einen Agonismustest (siehe Beispiel 1.2.1. der biologischen Beispiele) oder durch einen Antagonismustest (siehe Beispiel 1.2.2. der biologischen Beispiele) bestimmt werden, ob eine agonistische oder antagonistische Wirkung vorliegt.

Unter Antagonisten sind solche Moleküle zu verstehen, die an ihre entsprechenden Rezeptoren binden und welche die Initiierung des/der mit dem Rezeptor gekoppelten Signaltransduktionswege/s durch den oder die natürlich vorkommenden Liganden inhibieren. Üblicherweise kompetitieren die Antagonisten mit dem natürlich vorkommenden Liganden des Rezeptors um die Bindung an den Rezeptor. Aber auch andere Modifikationen des Rezeptors durch Moleküle, die verhindern, dass die mit dem Rezeptor gekoppelten Signaltransduktionswege durch den oder die natürlich vorkommenden Liganden aktiviert werden, sind möglich (z.B. nicht-kompetitive, sterische Modifikationen des Rezeptors).

Rezeptorantagonisten binden typischerweise selektiv an ihren bestimmten Rezeptor und nicht an andere Rezeptoren. Sie weisen normalerweise eine höhere Bindungsaffinität als der natürliche Ligand auf. Obwohl Antagonisten, die eine höhere Affinität zum Rezeptor haben als der natürliche Ligand, bevorzugt sind, können ebenfalls Antagonisten mit einer geringeren Affinität eingesetzt werden. Aber auch andere Modifikationen des Rezeptors durch Moleküle, die verhindern, das die mit dem Rezeptor gekoppelten Signaltransduktionswege durch den oder die natürlich vorkommenden Liganden aktiviert werden sind möglich (z.B. nicht-kompetitive, sterische Modifikationen des Rezeptors). Bevorzugterweise binden die Antagonisten reversibel an ihre korrespondierenden Rezeptoren.

Der EP₂ Rezeptor Antagonist hat eine bevorzugte Affinität für den EP₂ Rezeptor gegenüber jedem anderen EP-Rezeptor. Der Antagonismus wird in Gegenwart des natürlichen Agonisten gemessen (PGE₂).

Unter Agonisten sind solche Moleküle zu verstehen, die an ihre entsprechenden Rezeptoren binden und üblicherweise mit dem natürlich vorkommenden Liganden des Rezeptors um die Bindung an den Rezeptor kompetitieren und welche die Initiierung des mit dem Rezeptor gekoppelten Signaltransduktionsweges stimulieren. Agonisten können auch die Bindung des natürlichen Liganden unterstützen.

Rezeptoragonisten binden typischerweise selektiv an ihren bestimmten Rezeptor und nicht an andere Rezeptoren. Sie haben normalerweise eine höhere Bindungsaffinität als der natürliche Ligand. Obwohl Agonisten, die eine höhere Affinität zum Rezeptor haben als der natürliche Ligand, bevorzugt sind, können ebenfalls Agonisten mit einer geringeren Affinität eingesetzt werden. Bevorzugterweise binden die Agonisten reversibel an ihre korrespondierenden Rezeptoren.

Der EP₂ Rezeptor Agonist hat eine bevorzugte Affinität für den EP₂ Rezeptor gegenüber jedem anderen EP-Rezeptor.

Agonisten werden über die Initiierung der entsprechenden Rezeptor vermittelten Signaltransduktion und/oder physiologischen Wirkung getestet. Als Liganden werden die Verbindungen oder niedermolekulare Substanzen bezeichnet, die an einen Rezeptor binden. Ihre Bindung ist üblicherweise reversibel. Durch die Bindung eines Liganden an den entsprechenden Rezeptor wird der mit dem Rezeptor gekoppelte Signaltransduktionsweg aktiviert oder inaktiviert. Auf diese Art und Weise vermittelt der Ligand seine intrazelluläre Wirkung. Unter Liganden sind Agonisten und Antagonisten eines Rezeptors zu verstehen.

Die Substanz gemäß Beispiel 22 zeigt im zellulären Agonismustest keine Inhibition, im Antagonismustest jedoch eine gute Wirksamkeit (IC₅₀ = 0,11 x 10 E-6 M).
Ebenfalls Gegenstand der vorliegenden Erfindung ist die Verwendung der erfindungsgemäßen Substanzen als EP₂-Rezeptor Antagonisten für die Behandlung von Erkrankungen, die verursacht werden durch Störungen in der Signaltransduktionskette, an der der EP₂-Rezeptor beteiligt ist, wie beispielsweise Schmerz und Fertilitätsstörungen, und die ebenfalls geeignet sind für die Fertilitätskontrolle.

Im prä-ovulatorischen antralen Follikel ist die Eizelle von Kumulus Zellen umgeben, die einen dichten Zellkranz um die Eizelle bilden. Nach dem Peak des Lutenisierenden Hormons (LH-Peak) wird eine Reihe von Prozessen aktiviert, die eine starke morphologische Veränderung dieses Zellkranzes aus Kumulus Zellen zur Folge hat. Hierbei bilden die Kumulus Zellen eine extrazelluläre Matrix, die zur sogenannten Kumulus Expansion führt (Vanderhyden et al. Dev Biol. 1990 Aug;140(2):307-317). Diese Kumulus Expansion ist ein wichtiger Bestandteil des ovulatorischen Prozesses und der nachfolgenden Möglichkeit zur Fertilisation.

Bei der Kumulus Expansion sind Prostaglandine und hier das Prostaglandin E₂, dessen Synthese durch den LH-Peak induziert wird, von entscheidender Bedeutung. Prostanoid EP₂ Knock-out Mäuse (Hizaki et al.. Proc Natl Acad Sci U S A. 1999 Aug 31;96(18):10501-6.) zeigen eine deutlich verminderte Kumulus Expansion und starke Subfertilität, was die Bedeutung des Prostanoid EP₂-Rezeptors für diesen Prozess demonstriert.

Die erfindungsgemäßen Substanzen haben inhibitorische Wirkungen in Kumulus Expansionstests.
Gegenstand der vorliegenden Erfindung ist die Verwendung der erfindungsgemäßen Substanzen zur Fertilitätskontrolle.

Während der EP₂-Rezeptor Antagonist AH 6809 die Expansion des Kumulus erst bei einer Konzentration von 100 - 200 µM um nur ca. 30 % unterdrückt, kann in Anwesenheit von der Substanz gemäß Beispiel 22 eine 45 %ige Unterdrückung der Kumulus Expansion bereits bei einer 10 - 20fach geringeren Konzentration (10µM) erreicht werden. Bei diesen Versuchen kompetitieren die Testsubstanzen mit dem natürlichen EP₂-Rezeptor Agonisten PGE₂.

Gegenstand der vorliegenden Erfindung ist die Verwendung der erfindungsgemäßen Substanzen für die Hemmung der Kumulus Expansion und dadurch der Ovulation und Fertilisation zur Kontrazeption.

Prostaglandine spielen eine wichtige Rolle bei der Angiogenese (Sales, Jabbour, 2003, Reproduction 126, 559 - 567; Kuwano et al., 2004, FASEB J. 18, 300-310; Kamiyama et al., 2006, Oncogene 25, 7019-7028; Chang et al. 2005, Prostaglandins & other Lipid Mediators 76, 48-58).
Endometriose ist eine chronische Erkrankung, die verursacht wird durch Störungen der Blutgefäße. Circa 10 % der Frauen leiden regelmäßig an starken Blutungen während der Menstruation, verursacht durch Änderungen der Blutgefäße des Endometriums. Zusätzlich wurden strukturelle Unterschiede in den Blutgefäßen beobachtet, wie zum Beispiel die unvollständige Ausbildung der glatten Muskelzellschicht (Abberton et al., 1999, Hum. Reprod. 14, 1072-1079). Da der Blutverlust während der Menstruation zum Teil durch die Konstriktion der Blutgefäße geregelt wird, ist es naheliegend, dass die Defekte der glatten Muskulatur wesentlich zur Blutung beitragen.

Gegenstand der vorliegenden Erfindung ist die Verwendung der Substanzen der allgemeinen Formel I für die Behandlung von Endometriose.

Prostaglandine spielen eine wichtige Rolle bei der Uteruskontraktion, zu starke Kontraktionen sind verantwortlich für Menstruationsbeschwerden (Sales, Jabbour, 2003, Reproduction 126, 559 - 567).

Gegenstand der vorliegenden Erfindung ist die Verwendung der Substanzen der allgemeinen Formel I für die Behandlung von Menstruationsbeschwerden.

Zunehmende Forschungsergebnisse belegen auch die Bedeutung der EP-Rezeptoren, und insbesondere auch des EP₂-Rezeptoren bei einer Vielzahl von Krebsarten (z. B. Brustkrebs, Kolonkarzinom, Lungenkrebs, Prostatakrebs, Leukämie, Hautkrebs), was auf zukünftige Möglichkeiten des Einsatzes von Modulatoren (Antagonisten oder Agonisten) des EP2-Rezeptors für die Therapie und Vorbeugung (prophylaktisch und/oder adjuvant) von Krebs schließen lässt (Fulton et al. Cancer Res 2006; 66(20): 9794-7; Castellone et al. Science VOL 310 2005, 1504-1510; Chang et al. Cancer Res 2005; 65(11): 4496-9); Hull et al. Mol Cancer Ther 2004;3(8):1031-9; Richards et al. J Clin Endocrinol Metab 88: 2810-2816, 2003; Sinha et al. 2007, Cancer Res; 67(9):4507-13; Wang et al. 2004, Seminars in Oncology, Vol 31, No 1, Suppl 3: pp 64-73, Jain et al. Cancer Res 2006; 66(13): 6638-48)).

Gegenstand der vorliegenden Erfindung ist die Verwendung der Substanzen der allgemeinen Formel I für die Behandlung und Vorbeugung von Krebserkrankungen.

Prostaglandine spielen auch eine wichtige Rolle bei den Prozessen, die dem Knochenschwund entgegen wirken. Gegenstand der vorliegenden Erfindung ist daher die Verwendung der erfindungsgemäßen Substanzen für die Behandlung von Knochenschwund.
Reinold et al. (J. Clin. Invest. 115, 673-679 (2005)) beschreibt PGE₂ Rezeptoren vom EP₂-Subtyp als die Schlüssel-Signalelemente bei der inflammatorischen Hyperalgesie. Mäuse, die diesen Rezeptor nicht mehr tragen (EP₂^{-/-}), empfinden keinen spinalen inflammatorischen Schmerz. Es gibt Hinweise, dass eine inflammatorische, gesteigerte Schmerzempfindlichkeit behandelt werden kann, indem gezielt EP₂-Rezeptoren moduliert werden.

Gegenstand der vorliegenden Erfindung ist die Verwendung der erfindungsgemäßen Substanzen für die Behandlung von inflammatorischer Hyperalgesie.

Prostaglandine sind wichtige Mediatoren von entzündlichen Prozessen. Neuere Forschungsergebnisse zeigen die Beteiligung des EP2 Rezeptors bei entzündlichen Darmerkrankungen (e.g. Morbus Crohn): Sheibanie et al. The Journal of Immunology, 2007, 178: 8138-8147.

Gegenstand der vorliegenden Erfindung ist die Verwendung der erfindungsgemäßen Substanzen für die Behandlung von entzündlichen Erkrankungen, beispielsweise. entzündlichen Darmerkrankungen, wie Morbus Crohn.

Außerdem betrifft die Erfindung ein Verfahren zur Herstellung der erfindungsgemäßen Verbindungen der allgemeinen Formel I, das dadurch gekennzeichnet ist, dass man eine Verbindung der allgemeinen Formel Va bzw. Vb, 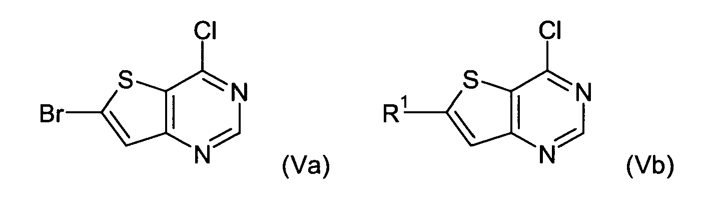 worin R¹ in Verbindung Vb die oben angegebene Bedeutung hat, mit einem Amin der allgemeinen Formel III, 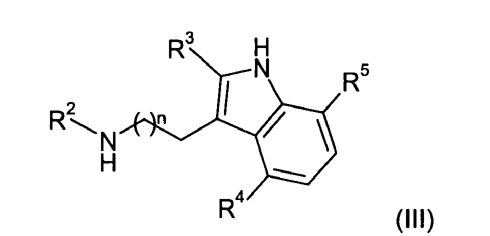 worin R², R³, R⁴ und R⁵ die oben angegebenen Bedeutungen haben nach dem Fachmann bekannten Methoden zu den erfindungsgemäßen Verbindungen der allgemeinen Formel I bzw. den Intermediaten der Formel II umgesetzt werden.

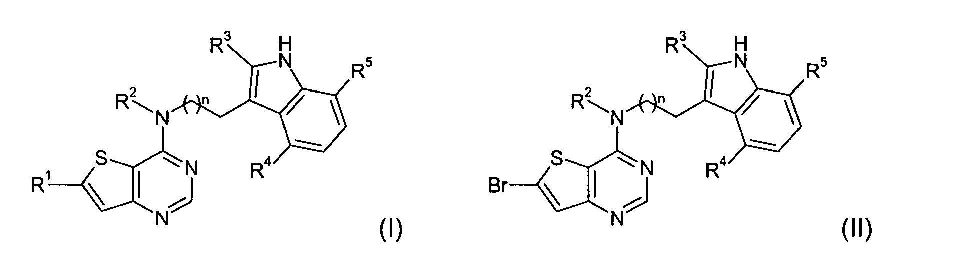

Die Intermediate der allgemeinen Formel II werden durch die Reaktion mit einer Boronsäure bzw. eines Boronsäureesters R¹B(OH)₂ der allgemeinen Formel IV, worin R¹ die oben angegebene Bedeutung hat in einer Palladium (0)-katalysierten Reaktion nach dem Fachmann bekannten Methoden zu weiteren erfindungsgemäßen Verbindungen der allgemeinen Formel I umgesetzt.

Die Umsetzung des Chlorthienopyrimidins der allgemeinen Formel Va bzw. Vb mit einem Amin der allgemeinen Formel III kann in einem inerten Lösungsmittel oder Lösungsmittelgemisch, wie beispielsweise N,N-Dimethylformamid, N,N-Dimethylacetamid, Toluol, n-Butanol, Tetrahydrofuran, NMP gegebenenfalls unter Zusatz einer Hilfsbase, wie beispielsweise N,N-Dimethylaminopyridin, Diisopropylethylamin, Triethylamin bei Temperaturen zwischen + 20 ° C und + 165 °C, vorzugsweise bei + 60 ° C bis + 120 °C erfolgen.

Eine weitere Möglichkeit besteht darin, die Umsetzung des Chlorthienopyrimidins der allgemeinen Formel Va bzw. Vb mit einem Amin der allgemeinen Formel III in einem inerten Lösungsmittel oder Lösungsmittelgemisch, wie beispielsweise N-Methylpyrolidinon, Toluol unter Palladium (0)-Katalyse (mit z.B. Pd(OAc)₂, Pd(PPh₃)₄, Pd₂(dba)₃, PdCl₂(dppf)) und Zusatz einer Base wie beispielsweise Natrium-tert.-butylat sowie eines geeigneten Liganden wie beispielsweise 2,2'-Bis(diphenylphosphino)-1,1'-binaphthyl bei Temperaturen zwischen +40°C und +150°C durchzuführen.

Die Umsetzung der aus der oben beschriebenen Reaktion des Thienopyrimidins Va mit einem Amin der allgemeinen Formel III erhaltenen Verbindungen der allgemeinen Formel II zu den Verbindungen der allgemeinen Formel I erfolgt beispielsweise durch Reaktion mit einer Boronsäure bzw. eines Boronsäureesters der allgemeinen Formel IV in einem Lösungsmittel oder Lösungsmittelgemisch, wie beispielsweise N-Methylpyrolidinon, Toluol, THF, Ethanol, n-Butanol, Wasser unter Palladium (0)-Katalyse (mit z.B. Pd(OAc)₂, Pd(PPh₃)₄, Pd₂(dba)₃, PdCl₂(dppf)) und Zusatz einer Base wie beispielsweise Natriumcarbonat, Kaliumcarbonat, Trietylamin sowie eines geeigneten Liganden Triphenylphosphin, Tri-o-Tolylphosphin bei Temperaturen zwischen +40°C und +150°C.

Die Herstellung der Salze erfolgt in üblicher Weise, indem man eine Lösung der Verbindung der Formel I mit der äquivalenten Menge oder einem Überschuss einer Base oder Säure, die gegebenenfalls in Lösung ist, versetzt und den Niederschlag abtrennt oder in üblicher Weise die Lösung aufarbeitet.

Die Erfindung betrifft damit auch Arzneimittel auf Basis der Verbindungen der allgemeinen Formel I und der üblichen Hilfs- oder Trägerstoffe.

Soweit die Herstellung der Ausgangsverbindungen nicht beschrieben wird, sind diese bekannt oder analog zu bekannten Verbindungen oder hier beschriebenen Verfahren herstellbar. Es ist ebenfalls möglich, alle hier beschriebenen Umsetzungen in Parallel-Reaktoren oder mittels kombinatorischer Arbeitstechniken durchzuführen.

Die erfindungsgemäßen Verbindungen der allgemeinen Formel I lassen sich wie in den Beispielen beschrieben herstellen.
Ausgehend von den Verbindungen der allgemeinen Formel Va-b lassen sich die Verbindungen der allgemeinen Formel I und II durch Umsetzung mit Aminen der allgemeinen Formel III nach dem Fachmann bekannten Verfahren herstellen. Ebenfalls lassen sich die erfindungungsgemäßen Verbindungen der allgemeinen Formel I durch Umsetzung von Verbindungen der allgemeinen Formel II mit Boronsäuren bzw. Boronsäureestern der allgemeinen Formel IV nach dem Fachmann bekannten Verfahren herstellen. Durch analoge Vorgehensweise unter Verwendung homologer Reagenzien zu den in den Beispielen beschriebenen Reagenzien lassen sich die weiteren Verbindungen der allgemeinen Formel I erhalten.

Die Substituenten am Rest R¹ der auf diese Weise erhaltenen Verbindungen der allgemeinen Formel I lassen sich nach dem Fachmann bekannten Methoden weiter zu vielfältigen funktionellen Gruppen und damit weiteren Verbindungen der allgemeinen Formel I umsetzen.

Zum Beispiel lässt sich ein Bromid oder Chlorid mittels Palladium(0)-katalysierter Reaktionen durch einen Aryl- bzw. Heteroarylring, ein substituiertes Alken bzw. Alkin, Amin oder eine Cyanogruppe ersetzen.

Eine Carboxyfunktion, Cyanogruppe oder ein Amin lässt sich beispielsweise nach dem Fachmann bekannten Methoden in Ester und Amide der allgemeinen Formel I überführen.

Ebenso lassen sich zum Beispiel Esterfunktionen bzw. eine Cyanogruppe in Verbindungen der allgemeinen Formel I nach Reduktion zum Aldehyd nach dem Fachmann bekannten Methoden in weitere Olefine bzw. mit Alkyl- oder Arylresten substituierte sekundäre Alkohole umgesetzen. Ebenfalls lässt sich eine Cyanogruppe in Verbindungen der allgemeinen Formel I nach dem Fachmann bekannten Methoden in mit Alkyl- oder Arylresten substituierte Ketone umsetzen, die sich dann zu den entsprechenden sekundären Alkoholen reduzieren lassen oder aber nach dem Fachmann bekannten Methoden in mit Alkyl- oder Arylresten substituierte tertiäre Alkohole überführt werden können.

Die Verbindung Va lässt sich ausgehend von der Verbindung VIII durch Cyclisierung zur Verbindung VIIa, Chlorierung zur Verbindung VI und anschließende Bromierung zur Verbindung Va nach dem Fachmann bekannten Verfahren herstellen. Die Verbindungen der allgemeinen Formel Vb lassen sich ausgehend von Verbindungen der allgemeinen Formel IX durch Cyclisierung zu Verbindungen der allgemeinen Formel VIIb und anschließende Chlorierung zu Verbindungen der allgemeinen Formel Vb nach dem Fachmann bekannten Verfahren herstellen.

Häufig verwendete Abkürzungen:
- NMP: N-Methylpyrrolidinon
- M: Molar
- TFA: Trifluoressigsäure
- ACN: Acetonitril
- eq.: Äquivalente
- THF: Tetrahydrofuran
- AC: Acetyl

Die nachfolgenden Beispiele dienen der näheren Erläuterung der Erfindung:

### Synthese des Thienopyrimidins Va ausgehend von Thiophen VIII 3H-Thieno[3,2-d]pyrimidin-4-on VIIa

Zu einer Lösung von 10g 3-Formylamino-thiophen-2-carbonsäuremethylester VIII in 12.8 ml Formamid gibt man 10.22g Ammoniumformiat und rührt 10 Stunden bei 140°C. Anschließend kühlt man auf 25°C ab, gibt Wasser hinzu und saugt den entstandenen Niederschlag ab. Wäscht mit Wasser nach und trocknen den Niederschlag im Vakuum. Man erhält auf diese Weise 5.74g Verbindung VIIa.
NMR (300 MHz, DMSO-d6): δ = 7.36 (1H), 8.11 (1H), 8.14 (1H), 12.43 (1H). *4-Chlor-thieno[3,2-d]pyrimidin VI*
Zu einer Lösung von 6.12ml Dimethylformamid in 45ml Methylenchlorid gibt man tropfenweise bei 25°C eine Lösung von 9.95ml Oxalylchlorid in 45ml Methylenchlorid zu. Anschließend werden 5.5 g Verbindung VIIa hinzugegeben und dann für 2.5 Stunden am Rückfluß erhitzt. Die Reaktionsmischung gibt man vorsichtig auf Wasser und extrahiert dreimal mit Methylenchlorid. Die vereinigten organischen Phasen werden über Natriumsulfat getrocknet und nach Filtration im Vakuum eingeengt. Nach dem Trocknen im Vakuum erhält man 4.9g Verbindung VI als Rohprodukt, dass ohne weitere Reinigung weiter umgesetzt wird.
NMR (300 MHz, DMSO-d6): δ = 7.73 (1H), 8.57 (1H), 9.01 (1H). *6-Brom-4-chlor-thieno[3,2-d]pyrimidin Va*
Zu einer Lösung von 10,32ml Lithiumdiisopropylamid (2M in Heptan/Tetrahydrofuran/ Ethylbenzol) in 35 ml THF tropft man langsam bei -78°C 2.95g Verbindung VI gelöst in 20ml THF zu und rührt 20 Minuten bei dieser Temperatur. Dann tropft man langsam 2.26ml 1,2-Dibrom-1,1,2,2-tetrafluorethan zu rührt zunächst 20 Minuten bei -78°C und dann weitere 2 Stunden bei 25°C. Die Reaktionsmischung wird in Wasser gegossen und dreimal mit Methylenchlorid extrahiert. Die vereinigten organischen Phasen werden über Natriumsulfat getrocknet und nach Filtration im Vakuum eingeengt. Der so erhaltene Rückstand wird durch Säulenchromatographie an Kieselgel mit Hexan / 0-50% Essigester gereinigt. Man erhält auf diese Weise 2.0g Verbindung Va.
NMR (300 MHz, DMSO-d6): δ = 8.04 (1H), 8.99 (1H).

### Beispielhafte Synthese eines Thienopyrimidins der allgemeinen Formel Vb ausgehend von einem Thiophen der allgemeinen Formel IX

### 6-Phenyl-3H-thieno[3,2-d]pyrimidin-4-on VIIb

Zu einer Lösung 50g 3-Amino-5-phenyl-thiophen-2-carbonsäuremethylester IX in 168ml Ameisensäure gibt man 21.4g Ammoniumacetat und erhitzt anschließend 6 Stunden unter Rückfluß.. Nach dem Abkühlen auf 25°C filtriert man und wäscht die erhaltenen Kristalle mit Wasser und trocknet anschließend im Vakuum. 62.3g des erhaltenen Formiats werden in 56.2 ml Formamid gegeben und mit 45.1g Ammoniumformiat versetzt. Man rührt 10 Stunden bei 140°C, kühlt auf 25°C ab, gibt Wasser hinzu und saugt den entstandenen Niederschlag ab. Es wird mit Wasser nachgewaschen und der Niederschlag im Vakuum getrocknet. Man erhält auf diese Weise 37.2g der Verbindung VIIb.
NMR (300 MHz, DMSO-d6): δ = 7.38-7.52 (3H), 7.78-7.86 (3H), 8.13 (1H).
*4-Chlor-6-phenyl-thieno[3,2-d]pyrimidin Vb*
5.0 g Verbindung VIIb werden in 15.3ml Phosphoroxychlorid 2 Stunden am Rückfluß erhitzt. Die Reaktionsmischung gibt man vorsichtig auf Eiswasser, stellt den pH-Wert mit verdünnter Natronlauge auf 10 ein und extrahiert dreimal mit Methylenchlorid. Die vereinigten organischen Phasen werden einmal mit Wasser gewaschen, über Natriumsulfat getrocknet und nach Filtration im Vakuum eingeengt. Den so erhaltenen Rückstand reinigt man durch Säulenchromatographie an Kieselgel mit Hexan / 0-40% Essigester. Man erhält auf diese Weise 3.8g Verbindung Vb.
NMR (300 MHz, DMSO-d6): δ = 7.48-7.57 (3H), 7.94- 8.00 (2H), 8.21 (1H), 8.99 (1H).

### Allgemeine Arbeitsvorschrift zur Synthese der Verbindungen der allgemeinen Formel I & II durch Aminierung der entsprechenden Thienopyrimidine Va-b

Die entsprechende Chlorverbindung Va-b wird 0.1 M in NMP vorgelegt, 2 eq Triethylamin und 2 eq des entsprechenden Amins III (c=0.5M in NMP) zugegeben und unter Rühren 30 min auf 170°C in der Mikrowelle erhitzt. Danach wird die Reaktionsmischung im Vakuum einrotiert und mittels Mitteldruckchromatographie an Kieselgel mit Hexan / 0-100% Essigester bzw. präparativer HPLC (Säule Purospher Star RP C18 4.6x125 5µm; Flussrate 1 ml/min; Eluenten A: 0.1% TFA in H₂O, B 0.1% TFA in ACN; Gradient jeweils bezogen auf B: 5% auf 95% (10') auf 95% (2') auf 5% (0.5') auf 5% (2.5'), MS: (M+H)⁺) gereinigt.
Nach dieser allgemeinen Reaktionsvorschrift wurden beispielsweise folgende Verbindungen synthetisiert: 1, 87-91.

### Allgemeine Arbeitsvorschrift zur Synthese der Thienopyrimidine I durch Suzuki Kupplung der Verbindungen II

Die entsprechende Bromverbindung II wird 0.2M in THF vorgelegt, 1.5 eq Boronsäure bzw. Boronsäureester IV, 3 eq Triethylamin (0.6M in THF), 7.5 mol% Pd(OAc)₂ (0.0375M in THF), 15mol% Tri-o-Tolylphosphin (0.05M in THF) und 100 eq Wasser zugegeben und für 30-40 min auf 120°C in der Mikrowelle erhitzt. Danach wird die Reaktionsmischung im Vakuum einrotiert und mittels Mitteldruckchromatographie an Kieselgel mit Hexan / 0-100% Essigester bzw. präparativer HPLC (Säule Purospher Star RP C18 4.6x125 5µm; Flussrate 1 ml/min; Eluenten A: 0.1 % TFA in H₂O, B 0.1 % TFA in ACN; Gradient jeweils bezogen auf B: 5% auf 95% (10') auf 95% (2') auf 5% (0.5') auf 5% (2.5'), MS: (M+H)⁺) gereinigt.
Nach dieser allgemeinen Reaktionsvorschrift wurden beispielsweise folgende Verbindungen synthetisiert: 2-86.

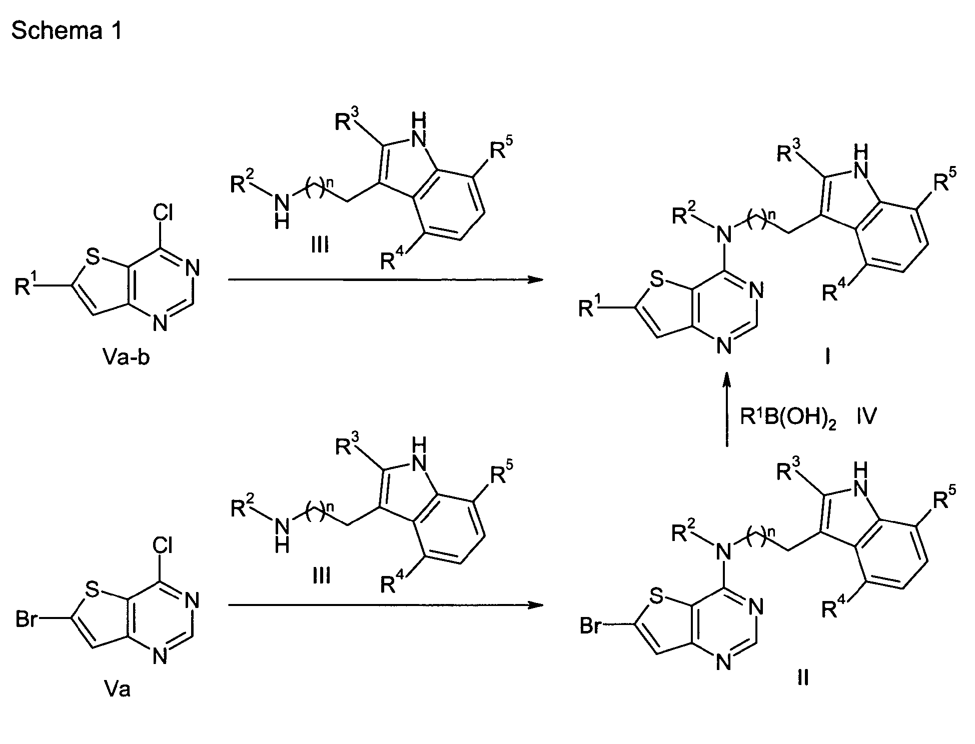

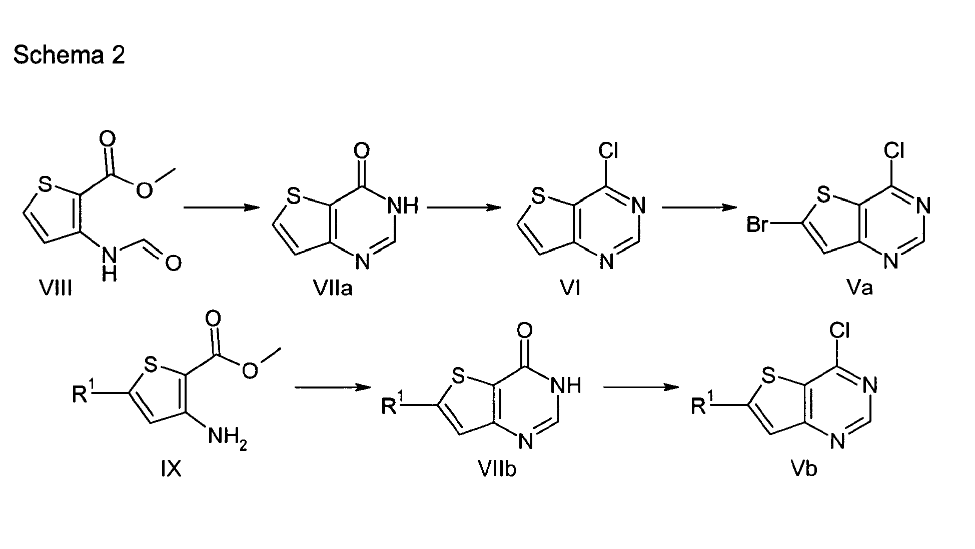

| Beispiel | Struktur | Retentionszeit | berechnete Masse | gefundene Masse |
|---|---|---|---|---|
| 1 | 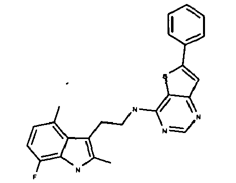 | 7,97 | 416 | 418 |
| 2 | 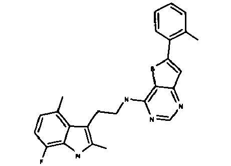 | 8,15 | 430 | 432 |
| 3 | 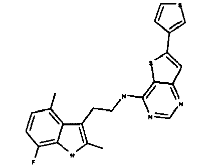 | 7,84 | 422 | 424 |
| 4 | 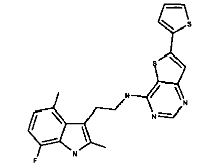 | 7,45 | 422 | 424 |
| 5 | 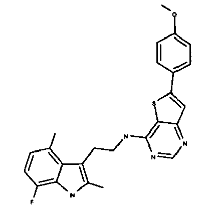 | 8,06 | 446 | 448 |
| 6 | 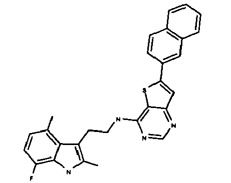 | 8,63 | 466 | 468 |
| 7 | 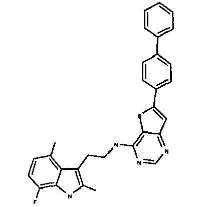 | 8,75 | 492 | 494 |
| 8 | 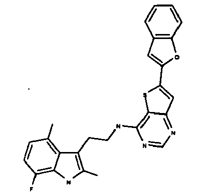 | 8,42 | 456 | 458 |
| 9 | 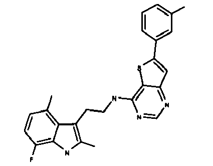 | 8,09 | 430 | 432 |
| 10 | 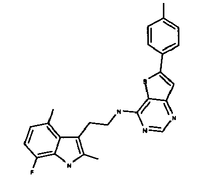 | 8,24 | 430 | 432 |
| 11 | 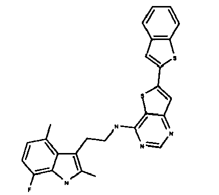 | 8,47 | 472 | 474 |
| 12 | 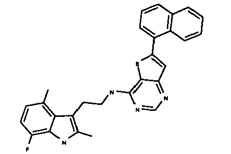 | 8,5 | 466 | 468 |
| 13 | 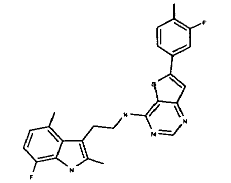 | 8,34 | 448 | 450 |
| 14 | 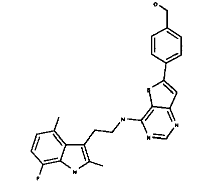 | 7,07 | 446 | 448 |
| 15 | 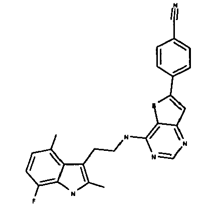 | 7,75 | 441 | 443 |
| 16 | 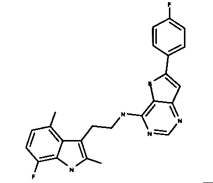 | 8 | 434 | 436 |
| 17 | 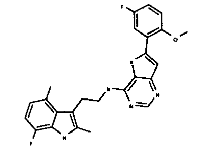 | 8,23 | 464 | 466 |
| 18 | 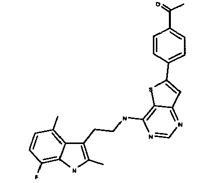 | 7,53 | 458 | 460 |
| 19 | 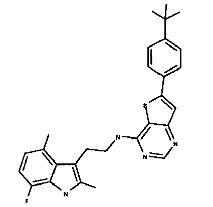 | 9,05 | 472 | 474 |
| 20 | 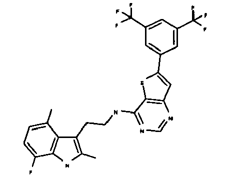 | 8,89 | 552 | 554 |
| 21 | 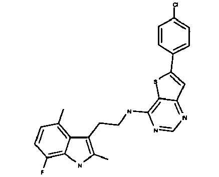 | 8,17 | 451 | 452 |
| 22 | 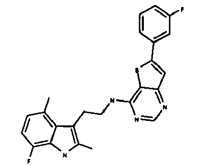 | 8,06 | 434 | 436 |
| 23 | 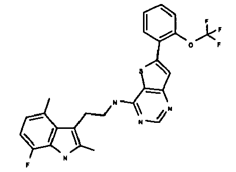 | 8,45 | 500 | 502 |
| 24 | 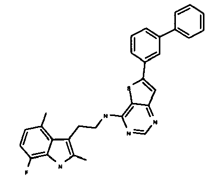 | 8,9 | 492 | 494 |
| 25 | 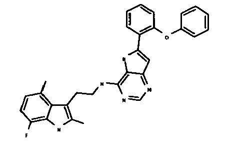 | 8,69 | 508 | 510 |
| 26 | 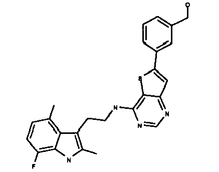 | 7,26 | 446 | 448 |
| 27 | 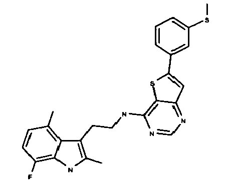 | 8,17 | 462 | 464 |
| 28 | 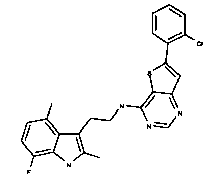 | 8,17 | 451 | 452 |
| 29 | 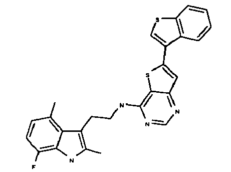 | 8,5 | 472 | 474 |
| 30 | 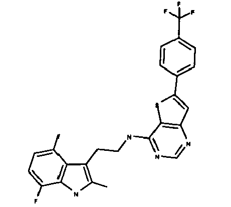 | 8,45 | 484 | 486 |
| 31 | 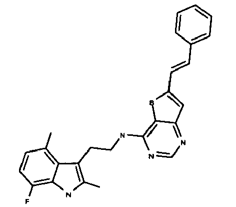 | 8,2 | 442 | 444 |
| 32 | 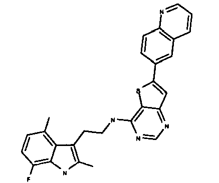 | 6,54 | 467 | 469 |
| 33 | 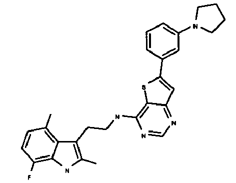 | 8,84 | 485 | 487 |
| 34 | 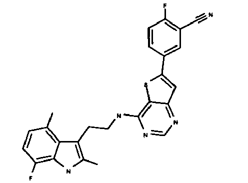 | 7,92 | 459 | 461 |
| 35 | 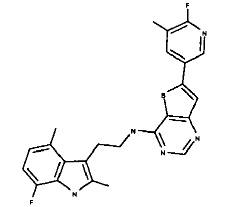 | 7,52 | 449 | 451 |
| 36 | 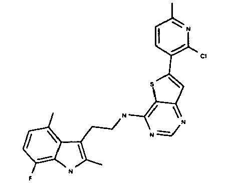 | 7,66 | 466 | 467 |
| 37 | 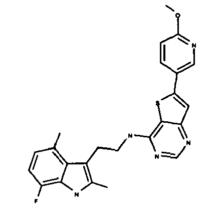 | 7,67 | 447 | 449 |
| 38 | 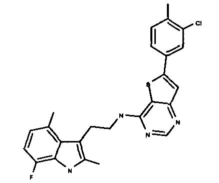 | 8,65 | 465 | 466 |
| 39 | 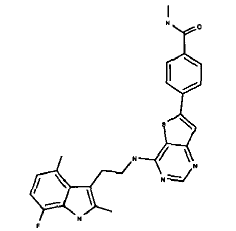 | 6,89 | 473 | 475 |
| 40 | 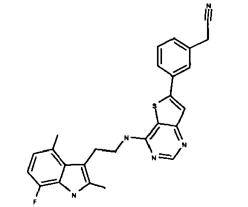 | 7,76 | 455 | 457 |
| 41 | 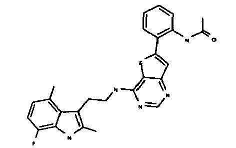 | 7 | 473 | 475 |
| 42 | 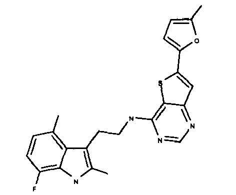 | 7,25 | 420 | 422 |
| 43 | 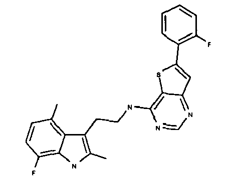 | 8,03 | 434 | 436 |
| 44 | 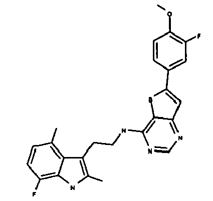 | 8,06 | 464 | 466 |
| 45 | 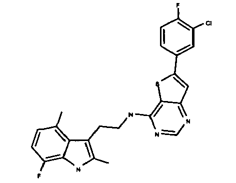 | 8,38 | 469 | 470 |
| 46 | 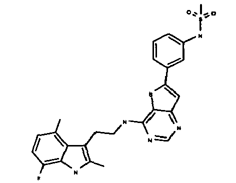 | 7,45 | 509 | 511 |
| 47 | 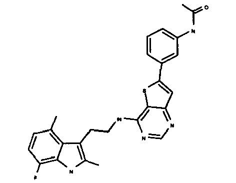 | 7,22 | 473 | 475 |
| 48 | 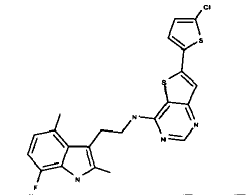 | 8,35 | 457 | 458 |
| 49 | 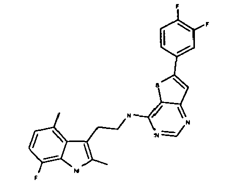 | 8,1 | 452 | 454 |
| 50 | 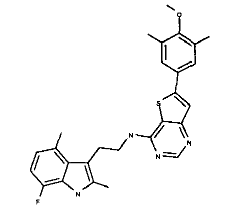 | 8,47 | 474 | 476 |
| 51 | 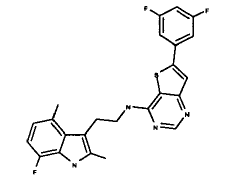 | 8 | 452 | 454 |
| 52 | 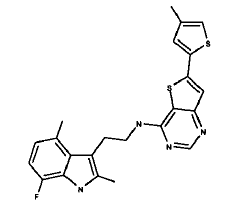 | 8,15 | 436 | 438 |
| 53 | 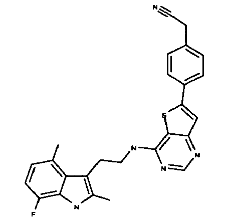 | 7,55 | 455 | 457 |
| 54 | 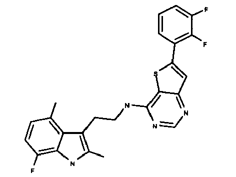 | 8,13 | 452 | 454 |
| 55 | 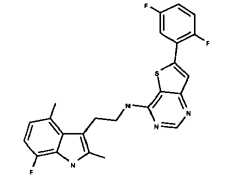 | 8,08 | 452 | 454 |
| 56 | 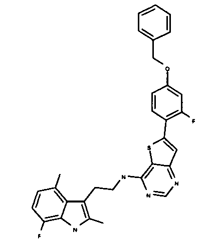 | 8,95 | 540 | 542 |
| 57 | 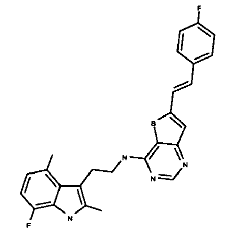 | 8,25 | 460 | 462 |
| 58 | 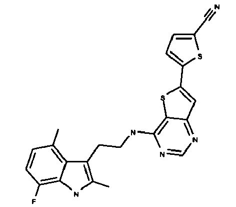 | 7,83 | 447 | 449 |
| 59 | 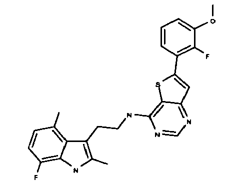 | 8,11 | 464 | 466 |
| 60 | 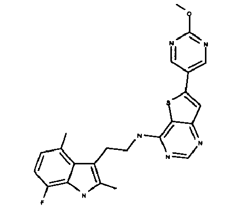 | 7,12 | 448 | 450 |
| 61 | 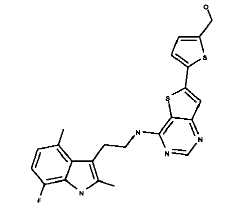 | 7,09 | 452 | 454 |
| 62 | 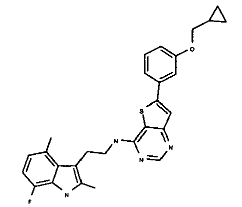 | 8,7 | 486 | 488 |
| 63 | 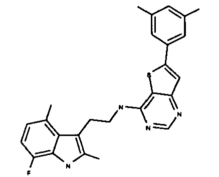 | 8,6 | 444 | 446 |
| 64 | 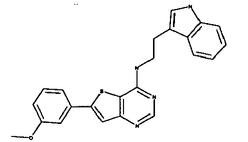 | 3,31 | 401 | 402 |
| 65 | 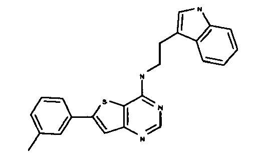 | 3,42 | 385 | 386 |
| 66 | 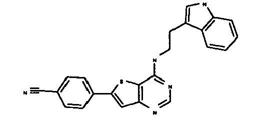 | 3,07 | 395 | 396 |
| 67 | 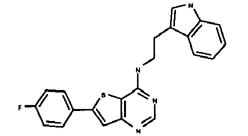 | 3,24 | 388 | 389 |
| 68 | 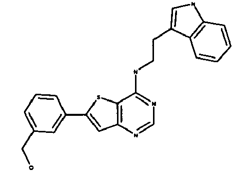 | 2,96 | 401 | 402 |
| 69 | 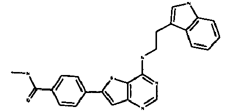 | 2,82 | 428 | 429 |
| 70 | 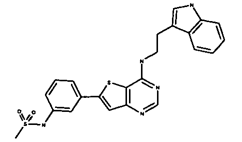 | 3,01 | 464 | 465 |
| 71 | 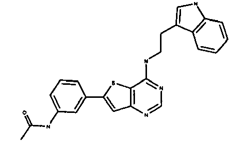 | 3,10 | 428 | 429 |
| 72 | 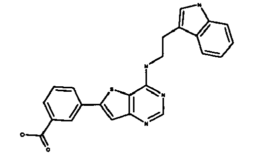 | 3,12 | 414 | 415 |
| 73 | 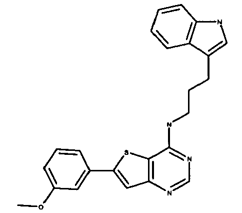 | 3,27 | 415 | 416 |
| 74 | 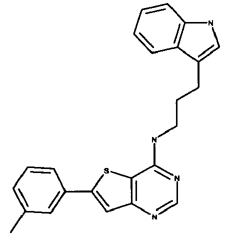 | 3,46 | 399 | 400 |
| 75 | 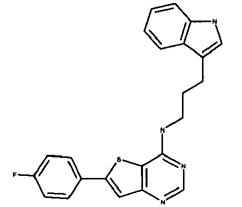 | 3,37 | 402 | 403 |
| 76 | 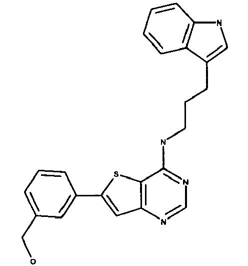 | 2,91 | 415 | 416 |
| 77 | 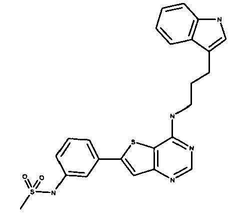 | 3,15 | 478 | 479 |
| 78 | 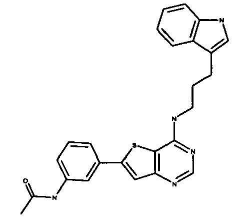 | 2,95 | 442 | 443 |
| 79 | 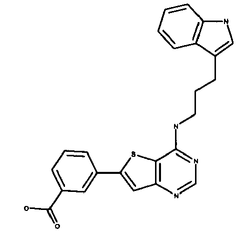 | 3,04 | 429 | 430 |
| 80 | 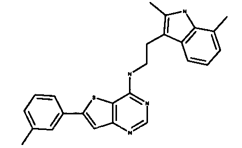 | 3,57 | 413 | 414 |
| 81 | 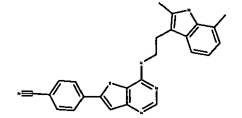 | 3,36 | 424 | 425 |
| 82 | 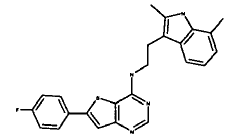 | 3,45 | 417 | 418 |
| 83 | 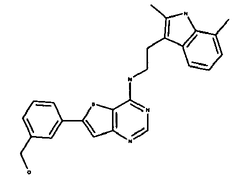 | 3,03 | 429 | 430 |
| 84 | 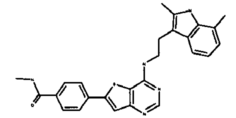 | 2,99 | 456 | 457 |
| 85 | 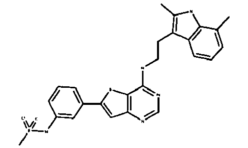 | 3,2 | 492 | 493 |
| 86 | 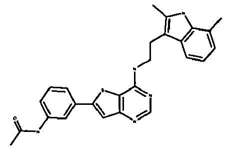 | 3,03 | 456 | 457 |

| Beispiel | Struktur | NMR (300 MHz, DMSO-d6) |
|---|---|---|
| 87 | 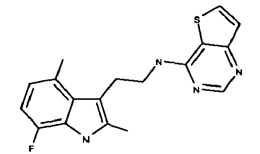 | δ = 2.26 (3H), 2.61 (3H), 3.03 (2H), 3.56 (2H), 6.53-6.67 (2H), 7.33 (1H), 8.01 (1H), 8.05 (1H), 8.41 (1H), 11.13 (1H) |
| 88 | 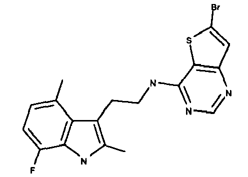 | δ = 2.24 (3H), 2.60 (3H), 3.01 (2H), 3.54 (2H), 6.53-6.67 (2H), 7.55 (1H), 8.08 (1H), 8.38 (1H), 11.13 (1H) |
| 89 | 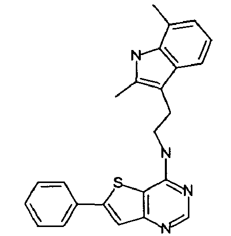 | δ = 2.27 (3H), 2.37 (3H), 2.94 (2H), 3.61 (2H), 6.74 (1H), 6.82 (1H), 7.30 (1H), 7.39-7.52 (3H), 7.78 (1H), 7.81 (2H), 7.95 (1H), 8.46 (1H), 10.56 (1H) |
| 90 | 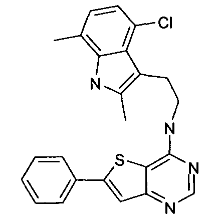 | δ = 2.23 (3H), 2.35 (3H), 3.15 (2H), 3.67 (2H), 6.72 (1H), 6.82 (1H), 7.37-7.53 (3H), 7.77 (1H), 7.80 (2H), 7.96 (1H), 8.41 (1H), 10.91 (1H) |
| 91 | 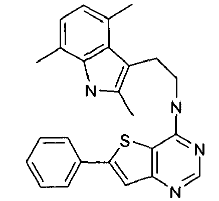 | δ = 2.29 (3H), 2.32 (3H), 2.62 (3H), 3.05 (2H), 3.56 (2H), 6.54 (1H), 6.61 (1H), 7.38-7.53 (3H), 7.78 (1H), 7.81 (2H), 8.03 (1H), 8.43 (1H), 10.52 (1H) |

### Biologische Beispiele:

### 1. Nachweis des Antagonismus des humanen Prostaglandin E₂ (Subtyp EP₂) Rezeptorsignals

### 1.1 Nachweisprinzip

Die Bindung von PGE₂ an den EP₂-Subtyp des humanen PGE₂-Rezeptors induziert die Aktivierung membranständiger Adenylatzyklasen und führt zur Bildung von cAMP. In Gegenwart des Phosphodiesteraseinhibitors IBMX wird das durch diese Stimulation akkumulierte und mittels Zell-Lyse freigesetzte cAMP in einem kompetitiven Nachweisverfahren eingesetzt. In diesem Test konkurriert das cAMP im Lysat mit cAMP-XL665 um die Bindung eines Eu-Kryptat markierten Anti-cAMP Antikörpers.
Dabei entsteht in Abwesenheit von zellulärem cAMP ein maximales Signal, welches auf der Kopplung dieses Antikörpers an das cAMP-XL665 Molekül beruht. Dadurch entsteht nach Anregung bei 337 nm ein auf FRET (fluorescence resonance energy transfer) basierendes, langanhaltendes Emissionssignal bei 665 nm (und bei 620 nM). Beide Signale werden in einem geeigneten Messgerät zeitlich versetzt, d.h. nach Abklingen der Hintergrundsfluoreszenz gemessen. Jegliche Erhöhung des durch Prostglandin-E₂-Gabe bedingten niedrigen FRET-Signals (gemessen als Well-Ratio-Veränderung = Emission₆₆₅ₙₘ/Emission₆₂₀ₙₘ * 10000) zeigt die Wirkung von Antagonisten.

### 1.2. Nachweisverfahren

### 1.2.1. Test auf Antagonismus (Angaben pro Vertiefung einer 384-Loch Platte):

Die in einer Testplatte und 30% DMSO vorgelegten Substanzlösungen (0.75µl) werden in 16 µl einer KRSB+IBMX-Stimulationslösung gelöst (1 X Krebs-Ringer Bicarbonate Buffer; Sigma-Aldrich # K-4002; inklusive 750 µM 3-Isobutyl-1-methylxanthine Sigma-Aldrich # I-7018), anschließend werden 15 µl davon in eine medienfreie Zellkulturplatte überführt, die kurz zuvor mit KRSB gewaschen worden war.
Nach einer 30-minütigen Vorinkubation bei Raumtemperatur (RT) werden 5 µl einer 4xPGE₂ Lösung (11 nM) zugegeben und in Gegenwart des Agonisten für weitere 60 min bei RT inkubiert (Volumen: ∼20 µl) bevor die Reaktion anschließend durch Zugabe von 5 µl Lysispuffer gestoppt und für weitere 20 min bei RT inkubiert wird (Volumen: ∼25 µl). Das Zell-Lysat wird anschließend in eine Messplatte überführt und entsprechend den Herstellerangaben (cyclic AMP kit Cisbio International # 62AMPPEC) gemessen.

### 1.2.2. Test auf Agonismus (Angaben pro Vertiefung einer 384-Loch Platte):

Die in einer in einer Testplatte und 30% DMSO vorgelegten Substanzlösungen (0.75µl) werden in 16 µl einer KRSB+IBMX-Stimulationslösung gelöst (1 X Krebs-Ringer Bicarbonate Buffer; Sigma-Aldrich # K-4002; inklusive 750 µM 3-Isobutyl-1-methylxanthine Sigma-Aldrich # I-7018), anschließend werden 15 µl davon in eine medienfreie Zellkulturplatte überführt, die kurz zuvor mit KRSB gewaschen worden war.
Nach einer 60-minütigen Inkubation bei Raumtemperatur (RT; Volumen: ∼15 µl) wird die Reaktion anschließend durch Zugabe von 5 µl Lysisbuffer gestoppt und für weitere 20 min bei RT inkubiert (Volumen: ∼20 µl). Das Zell-Lysat wird anschließend in eine Messplatte überführt und entsprechend den Herstellerangaben (cyclic AMP kit Cisbio International # 62AMPPEC) gemessen.

### 2. Der EP₂-Subtyp des PGE₂-Rezeptors und die prä-ovulatorische Kumulus Expansion

### 2.1. Hintergrund:

Im prä-ovulatorischen antralen Follikel ist die Eizelle von Kumulus Zellen umgeben, die einen dichten Zellkranz um die Eizelle bilden. Nach dem LH-Peak (Lutenisierendes Hormon) wird eine Reihe von Prozessen aktiviert, die eine starke morphologische Veränderung dieses Zellkranzes aus Kumulus Zellen zur Folge hat. Hierbei bilden die Kumulus Zellen eine extrazelluläre Matrix, die zur sogenannten Kumulus Expansion führt (Vanderhyden et al. Dev Biol. 1990 Aug;140(2):307-317). Diese Kumulus Expansion ist ein wichtiger Bestandteil des ovulatorischen Prozesses und der nachfolgenden Möglichkeit zur Fertilisation.

Bei der Kumulus Expansion sind Prostaglandine und hier das Prostaglandin E₂, dessen Synthese durch den LH-Peak induziert wird, von entscheidender Bedeutung. Prostanoid EP₂ Knock-out Mäuse (Hizaki et al. Proc Natl Acad Sci U S A. 1999 Aug 31;96(18):10501-6.) zeigen eine deutlich verminderte Kumulus Expansion und starke Subfertilität, was die Bedeutung des Prostanoid EP₂-Rezeptors für diesen Prozess demonstriert.

### 2.2. Kumulus Expansions Test in vitro

In immaturen weiblichen Mäusen wird in einem Alter von 14-18 Tagen die Follikulogenese durch eine einmalige Gabe (intraperitoneal) von 10 I.E. PMSG (Pregnant Mare Serum Gonadotropine; Sigma G-4877, Lot 68H0909) induziert. 47-50 Stunden nach der Injektion werden die Ovarien entnommen und die Kumulus-Eizell Komplexe entnommen. Der Kumulus Komplex ist in diesem Stadium noch nicht expandiert.
Die Kumulus-Eizell Komplexe werden nun für 20-24 Stunden mit Prostaglandin E₂ (PGE₂) (0,3 µM), Vehikel Kontrolle (Ethanol) oder Testsubstanzen inkubiert. Medium: alpha- MEM Medium mit 0,1 mM IBMX, Pyruvate (0,23 mM) Glutamine (2 mM), Pen/Strep 100 IU/ml Pen. und 100 µg/ml Strep.) und HSA (8 mg/ml)). Danach wird die Kumulus Expansion durch die Einteilung in vier Stadien (nach Vanderhyden et al. Dev Biol. 1990 Aug;140(2):307-317) festgestellt.

**Tabelle 1: Beispiel für die biologische Wirksamkeit der erfindungsgemäßen Verbindungen (gemessen mittels cAMP Antagonismustest):**

| Substanz gemäß Beispiel | Antagonismus [IC₅₀, µM] |
|---|---|
| 22 | 0,111 |
| 30 | 0,101 |
| 55 | 0,097 |

## Patentansprüche

1. Verbindungen der allgemeinen Formel I, 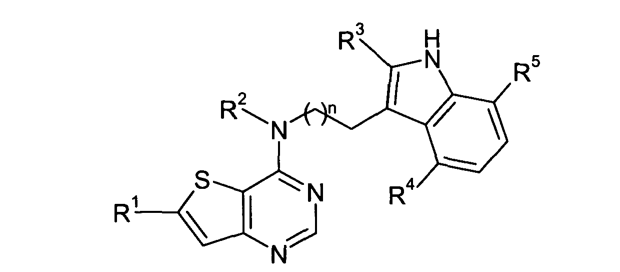 wobei
R¹ ein Wasserstoff, Halogen, eine C₁-C₄-Alkylgruppe,
eine CH=CH-Arylgruppe mit einem 6-10-gliedrigen, mono- oder bicyclischen Arylring, der jeweils unsubstituiert oder gegebenenfalls ein- bis dreifach substituiert ist,
eine CH=CH-Heteroarylgruppe mit einem 5-10-gliedrigen, mono- oder bicyclischen Heteroarylring, der jeweils unsubstituiert oder gegebenenfalls ein- bis dreifach substituiert ist,
ein 6-10-gliedriger, mono- oder bicyclischer Arylring, der jeweils unsubstituiert oder gegebenenfalls ein- bis dreifach substituiert ist, ein 5-10-gliedriger, mono- oder bicyclischer Heteroarylring, der jeweils unsubstituiert oder gegebenenfalls ein- bis dreifach substituiert ist,
wobei die Substituenten jeweils ausgewählt sein können aus der Gruppe
Halogen, Cyano, CH₂CN, R⁶, OR⁶, CH₂OR⁶, OC(O)R⁶, S(O)ₙR⁶, wobei n = 0, 1, 2 bedeutet,
SO₂NHR⁶, SO₂NHC(O)R⁶, NR⁶R⁷, NHC(O)R⁶, NHSO₂R⁶, CH₂NR⁶R⁷, CH₂NHC(O)R⁶, C(OH)R⁶R⁷, C(O)R⁶, CO₂R⁶, C(O)NR⁶R⁷,
n 1 oder 2,
R² ein Wasserstoff, eine C₁-C₄-Alkylgruppe,
R³ ein Wasserstoff, eine C₁-C₄-Alkylgruppe,
R⁴ ein Wasserstoff, Halogen, Cyano, eine C₁-C₄-Alkylgruppe,
R⁵ ein Wasserstoff, Halogen, Cyano, eine C₁-C₄-Alkylgruppe,
R⁶ ein Wasserstoff, eine C₁-C₄-Alkylgruppe, eine C₂-C₄-Alkenylgruppe, eine C₂-C₄-Alkinylgruppe, eine C₃-C₆-Cycloalkylgruppe, eine CH₂-C₃-C₆-Cycloalkylgruppe, ein 6-gliedriger Arylring, ein 5-6-gliedriger Heteroarylring oder eine CH₂-Aryl- bzw. Heteroarylgruppe, wobei der Arylrest 6-gliedrig und der Heteroarylrest 5 bzw. 6-gliedrig ist,
R⁷ ein Wasserstoff, eine C₁-C₄-Alkylgruppe,
R⁶, R⁷ zusammen einen 3-6-gliedrigen Ring bilden,
bedeuten, sowie deren Stereoisomere, Diastereomere, Enantiomere, Salze und deren Cyclodextrinclathraten.

2. Verbindungen gemäß Anspruch 1, wobei
R¹ ein Wasserstoff, Halogen, eine C₁-C₄-Alkylgruppe,
eine CH=CH-Arylgruppe mit einem 6-10-gliedrigen, mono- oder bicyclischen Arylring, der jeweils unsubstituiert oder gegebenenfalls ein- bis dreifach substituiert ist,
eine CH=CH-Heteroarylgruppe mit einem 5-10-gliedrigen, mono- oder bicyclischen Heteroarylring, der jeweils unsubstituiert oder gegebenenfalls ein- bis dreifach substituiert ist,
ein 6-10-gliedriger, mono- oder bicyclischer Arylring, der jeweils unsubstituiert oder gegebenenfalls ein- bis dreifach substituiert ist, ein 5-10-gliedriger, mono- oder bicyclischer Heteroarylring, der
jeweils unsubstituiert oder gegebenenfalls ein- bis dreifach substituiert ist,
wobei die Substituenten jeweils ausgewählt sein können aus der Gruppe
Halogen, Cyano, CH₂CN, R⁶, OR⁶, CH₂OR⁶, OC(O)R⁶, S(O)ₙR⁶, wobei n = 0, 1, 2 bedeutet,
SO₂NHR⁶, SO₂NHC(O)R⁶, NR⁶R⁷, NHC(O)R⁶, NHSO₂R⁶, CH₂NR⁶R⁷, CH₂NHC(O)R⁶, C(OH)R⁶R⁷, C(O)R⁶, CO₂R⁶, C(O)NR⁶R⁷,
n 1 oder 2,
R² ein Wasserstoff, eine Methylgruppe,
R³ ein Wasserstoff, eine C₁-Alkylgruppe,
R⁴ ein Wasserstoff, Halogen, Cyano, eine C₁-C₄-Alkylgruppe,
R⁵ ein Wasserstoff, Halogen, Cyano, eine C₁-C₄-Alkylgruppe,
R⁶ ein Wasserstoff, eine C₁-C₄-Alkylgruppe, eine C₂-C₄-Alkenylgruppe, eine C₂-C₄-Alkinylgruppe, eine C₃-C₆-Cycloalkylgruppe, eine CH₂-C₃-C₆-Cycloalkylgruppe, ein 6-gliedriger Arylring, ein 5-6-gliedriger Heteroarylring oder eine CH₂-Aryl- bzw. Heteroarylgruppe, wobei der Arylrest 6-gliedrig und der Heteroarylrest 5 bzw. 6-gliedrig ist,
R⁷ ein Wasserstoff, eine C₁-C₄-Alkylgruppe,
R⁶, R⁷ zusammen einen 3-6-gliedrigen Ring bilden,
bedeuten.

3. Verbindungen gemäß Anspruch 1-2, wobei
R¹ ein Wasserstoff, Halogen,
eine CH=CH-Arylgruppe mit einem 6-10-gliedrigen, mono- oder bicyclischen Arylring, der jeweils unsubstituiert oder gegebenenfalls ein- bis dreifach substituiert ist,
eine CH=CH-Heteroarylgruppe mit einem 5-10-gliedrigen, mono- oder bicyclischen Heteroarylring, der jeweils unsubstituiert oder gegebenenfalls ein- bis dreifach substituiert ist,
ein 6-10-gliedriger, mono- oder bicyclischer Arylring, der jeweils unsubstituiert oder gegebenenfalls ein- bis dreifach substituiert ist, ein 5-10-gliedriger, mono- oder bicyclischer Heteroarylring, der jeweils unsubstituiert oder gegebenenfalls ein- bis dreifach substituiert ist,
wobei die Substituenten jeweils ausgewählt sein können aus der Gruppe
Halogen, Cyano, CH₂CN, R⁶, OR⁶, CH₂OR⁶, OC(O)R⁶, S(O)ₙR⁶, wobei n = 0, 1, 2 bedeutet,
SO₂NHR⁶, SO₂NHC(O)R⁶, NR⁶R⁷, NHC(O)R⁶, NHSO₂R⁶, CH₂NR⁶R⁷, CH₂NHC(O)R⁶, C(OH)R⁶R⁷, C(O)R⁶, CO₂R⁶, C(O)NR⁶R⁷,
n 1 oder 2,
R² ein Wasserstoff, eine Methylgruppe,
R³ ein Wasserstoff, eine C₁-Alkylgruppe,
R⁴ ein Wasserstoff, Halogen, Cyano, eine C₁-C₄-Alkylgruppe,
R⁵ ein Wasserstoff, Halogen, Cyano, eine C₁-C₄-Alkylgruppe,
R⁶ ein Wasserstoff, eine C₁-C₄-Alkylgruppe, eine C₂-C₄-Alkenylgruppe, eine C₂-C₄-Alkinylgruppe, eine C₃-C₆-Cycloalkylgruppe, eine CH₂-C₃-C₆-Cycloalkylgruppe, ein 6-gliedriger Arylring, ein 5-6-gliedriger Heteroarylring oder eine CH₂-Aryl- bzw. Heteroarylgruppe, wobei der Arylrest 6-gliedrig und der Heteroarylrest 5 bzw. 6-gliedrig ist,
R⁷ ein Wasserstoff, eine C₁-C₄-Alkylgruppe,
R⁶, R⁷ zusammen einen 3-6-gliedrigen Ring bilden,
bedeuten.

4. Verbindungen gemäß Anspruch 1-3, wobei
R¹ ein Wasserstoff, Halogen,
eine CH=CH-Phenylgruppe, wobei der Phenylring jeweils unsubstituiert oder gegebenenfalls ein- bis dreifach substituiert ist, eine CH=CH-Heteroarylgruppe mit einem 5-6-gliedrigen Heteroarylring, der jeweils unsubstituiert oder gegebenenfalls ein- bis dreifach substituiert ist,
ein 6-10-gliedriger, mono- oder bicyclischer Arylring, der jeweils unsubstituiert oder gegebenenfalls ein- bis dreifach substituiert ist, ein 5-10-gliedriger, mono- oder bicyclischer Heteroarylring, der jeweils unsubstituiert oder gegebenenfalls ein- bis dreifach substituiert ist,
wobei die Substituenten jeweils ausgewählt sein können aus der Gruppe
Halogen, Cyano, CH₂CN, R⁶, OR⁶, CH₂OR⁶, OC(O)R⁶, S(O)ₙR⁶, wobei n = 0, 1, 2 bedeutet,
SO₂NHR⁶, ,SO₂NHC(O)R⁶, NR⁶R⁷, NHC(O)R⁶, NHSO₂R⁶, CH₂NR⁶R⁷, CH₂NHC(O)R⁶, C(OH)R⁶R⁷, C(O)R⁶, CO₂R⁶, C(O)NR⁶R⁷,
n 1 oder 2,
R² ein Wasserstoff, eine Methylgruppe,
R³ ein Wasserstoff, eine C₁-Alkylgruppe,
R⁴ ein Wasserstoff, Halogen, Cyano, eine C₁-C₄-Alkylgruppe,
R⁵ ein Wasserstoff, Halogen, Cyano, eine C₁-C₄-Alkylgruppe,
R⁶ ein Wasserstoff, eine C₁-C₄-Alkylgruppe, eine C₂-C₄-Alkenylgruppe, eine C₂-C₄-Alkinylgruppe, eine C₃-C₆-Cycloalkylgruppe, eine CH₂-C₃-C₆-Cycloalkylgruppe, ein 6-gliedriger Arylring, ein 5-6-gliedriger Heteroarylring oder eine CH₂-Aryl- bzw. Heteroarylgruppe, wobei der Arylrest 6-gliedrig und der Heteroarylrest 5 bzw. 6-gliedrig ist,
R⁷ ein Wasserstoff, eine C₁-C₄-Alkylgruppe,
R⁶, R⁷ zusammen einen 3-6-gliedrigen Ring bilden,
bedeuten.

5. Verbindungen gemäß den vorangegangenen Ansprüchen ausgewählt aus einer Gruppe, die die folgenden Verbindungen enthält:
1. [2-(7-Fluor-2,4-dimethyl-1H-indol-3-yl)-ethyl]-(6-phenyl-thieno[3,2-d]pyrimidin-4-yl)-amin
2. [2-(7-Fluor-2,4-dimethyl-1H-indol-3-yl)-ethyl]-(6-o-tolyl-thieno[3,2-d]pyrimidin-4-yl)-amin
3. [2-(7-Fluor-2,4-dimethyl-1H-indol-3-yl)-ethyl]-(6-thiophen-3-yl-thieno[3,2-d]pyrimidin-4-yl)-amin
4. 2-(7-Fluor-2,4-dimethyl-1H-indol-3-yl)-ethyl]-(6-thiophen-2-yl-thieno[3,2-d]pyrimidin-4-yl)-amin
5. [2-(7-Fluor-2,4-dimethyl-1H-indol-3-yl)-ethyl]-[6-(4-methoxy-phenyl)-thieno[3,2-d]pyrimidin-4-yl]-amin
6. 2-(7-Fluor-2,4-dimethyl-1H-indol-3-yl)-ethyl]-(6-naphthalen-2-yl-thieno[3,2-d]pyrimidin-4-yl)-amin
7. (6-Biphenyl-4-yl-thieno[3,2-d]pyrimidin-4-yl)-[2-(7-fluor-2,4-dimethyl-1H-indol-3-yl)-ethyl]-amin
8. (6-Benzofuran-2-yl-thieno[3,2-d]pyrimidin-4-yl)-[2-(7-fluor-2,4-dimethyl-1H-indol-3-yl)-ethyl]-amin
9. [2-(7-Fluor-2,4-dimethyl-1H-indol-3-yl)-ethyl]-(6-m-tolyl-thieno[3,2-d]pyrimidin-4-yl)-amin
10. [2-(7-Fluor-2,4-dimethyl-1H-indol-3-yl)-ethyl]-(6-p-tolyl-thieno[3,2-d]pyrimidin-4-yl)-amin
11. (6-Benzo[b]thiophen-2-yl-thieno[3,2-d]pyrimidin-4-yl)-[2-(7-fluor-2,4-dimethyl-1H-indol-3-yl)-ethyl]-amin
12. [2-(7-Fluor-2,4-dimethyl-1H-indol-3-yl)-ethyl]-(6-naphthalen-1-yl-thieno[3,2-d]pyrimidin-4-yl)-amin
13. [2-(7-Fluor-2,4-dimethyl-1H-indol-3-yl)-ethyl]-[6-(3-fluor-4-methyl-phenyl)-thieno[3,2-d]pyrimidin-4-yl]-amin
14. (4-{4-[2-(7-Fluor-2,4-dimethyl-1H-indol-3-yl)-ethylamino]-thieno[3,2-d]pyrimidin-6-yl}-phenyl)-methanol
15.4-{4-[2-(7-Fluor-2,4-dimethyl-1H-indol-3-yl)-ethylamino]-thieno[3,2-d]pyrimidin-6-yl}-benzonitril
16. [2-(7-Fluor-2,4-dimethyl-1H-indol-3-yl)-ethyl]-[6-(4-fluor-phenyl)-thieno[3,2-d]pyrimidin-4-yl]-amin
17. [2-(7-Fluor-2,4-dimethyl-1H-indol-3-yl)-ethyl]-[6-(5-fluor-2-methoxyphenyl)-thieno[3,2-d]pyrimidin-4-yl]-amin
18.1-(4-{4-[2-(7-Fluor-2,4-dimethyl-1H-indol-3-yl)-ethylamino]-thieno[3,2-d]pyrimidin-6-yl}-phenyl)-ethanon
19.[6-(4-tert-Butyl-phenyl)-thieno[3,2-d]pyrimidin-4-yl]-[2-(7-fluor-2,4-dimethyl-1H-indol-3-yl)-ethyl]-amin
20. [6-(3,5-Bis-trifluormethyl-phenyl)-thieno[3,2-d]pyrimidin-4-yl]-[2-(7-fluor-2,4-dimethyl-1H-indol-3-yl)-ethyl]-amin
21.[6-(4-Chlor-phenyl)-thieno[3,2-d]pyrimidin-4-yl]-[2-(7-fluor-2,4-dimethyl-1H-indol-3-yl)-ethyl]-amin
22.[2-(7-Fluor-2,4-dimethyl-1H-indol-3-yl)-ethyl]-[6-(3-fluor-phenyl)-thieno[3,2-d]pyrimidin-4-yl]-amin
23.[2-(7-Fluor-2,4-dimethyl-1H-indol-3-yl)-ethyl]-[6-(2-trifluormethoxyphenyl)-thieno[3,2-d]pyrimidin-4-yl]-amin
24.(6-Biphenyl-3-yl-thieno[3,2-d]pyrimidin-4-yl)-[2-(7-fluor-2,4-dimethyl-1H-indol-3-yl)-ethyl]-amin
25.[2-(7-Fluor-2,4-dimethyl-1H-indol-3-yl)-ethyl]-[6-(2-phenoxy-phenyl)-thieno[3,2-d]pyrimidin-4-yl]-amin
26.(3-{4-[2-(7-Fluor-2,4-dimethyl-1H-indol-3-yl)-ethylamino]-thieno[3,2-d]pyrimidin-6-yl}-phenyl)-methanol
27.[2-(7-Fluor-2,4-dimethyl-1H-indol-3-yl)-ethyl]-[6-(3-methylsulfanyl-phenyl)-thieno[3,2-d]pyrimidin-4-yl]-amin
28. [6-(2-Chlor-phenyl)-thieno[3,2-d]pyrimidin-4-yl]-[2-(7-fluor-2,4-dimethyl-1H-indol-3-yl)-ethyl]-amin
29. (6-Benzo[b]thiophen-3-yl-thieno[3,2-d]pyrimidin-4-yl)-[2-(7-fluor-2,4-dimethyl-1H-indol-3-yl)-ethyl]-amin
30.[2-(7-Fluor-2,4-dimethyl-1H-indol-3-yl)-ethyl]-[6-(4-trifluormethyl-phenyl)-thieno[3,2-d]pyrimidin-4-yl]-amin
31. [2-(7-Fluor-2,4-dimethyl-1H-indol-3-yl)-ethyl]-[6-((E)-styryl)-thieno[3,2-d]pyrimidin-4-yl]-amin
32.[2-(7-Fluor-2,4-dimethyl-1H-indol-3-yl)-ethyl]-(6-chinolin-6-yl-thieno[3,2-d]pyrimidin-4-yl)-amin
33. [2-(7-Fluor-2,4-dimethyl-1H-indol-3-yl)-ethyl]-[6-(3-pyrrolidin-1-yl-phenyl)-thieno[3,2-d]pyrimidin-4-yl]-amin
34.2-Fluor-5-{4-[2-(7-fluor-2,4-dimethyl-1H-indol-3-yl)-ethylaminoJ-thieno[3,2-d]pyrimidin-6-yl}-benzonitril
35.[2-(7-Fluor-2,4-dimethyl-1H-indol-3-yl)-ethyl]-[6-(6-fluor-5-methyl-pyridin-3-yl)-thieno[3,2-d]pyrimidin-4-yl]-amin
36.[6-(2-Chlor-6-methyl-pyridin-3-yl)-thieno[3,2-d]pyrimidin-4-yl]-[2-(7-fluor-2,4-dimethyl-1H-indol-3-yl)-ethyl]-amin
37.[2-(7-Fluor-2,4-dimethyl-1H-indol-3-yl)-ethyl]-[6-(6-methoxy-pyridin-3-yl)-thieno[3,2-d]pyrimidin-4-yl]-amin
38.[6-(3-Chlor-4-methyl-phenyl)-thieno[3,2-d]pyrimidin-4-yl]-[2-(7-fluor-2,4-dimethyl-1H-indol-3-yl)-ethyl]-amin
39.4-{4-[2-(7-Fluor-2,4-dimethyl-1H-indol-3-yl)-ethylamino]-thieno[3,2-d]pyrimidin-6-yl}-N-methyl-benzamid
40.(3-{4-[2-(7-Fluor-2,4-dimethyl-1H-indol-3-yl)-ethylamino]-thieno[3,2-d]pyrimidin-6-yl}-phenyl)-acetonitril
41. N-(2-{4-[2-(7-Fluor-2,4-dimethyl-1H-indol-3-yl)-ethylamino]-thieno[3,2-d]pyrimidin-6-yl}-phenyl)-acetamid
42.[2-(7-Fluor-2,4-dimethyl-1H-indol-3-yl)-ethyl]-[6-(5-methyl-furan-2-yl)-thieno[3,2-d]pyrimidin-4-yl]-amin
43.[2-(7-Fluor-2,4-dimethyl-1H-indol-3-yl)-ethyl]-[6-(2-fluor-phenyl)-thieno[3,2-d]pyrimidin-4-yl]-amin
44.[2-(7-Fluor-2,4-dimethyl-1H-indol-3-yl)-ethyl]-[6-(3-fluor-4-methoxyphenyl)-thieno[3,2-d]pyrimidin-4-yl]-amin
45. [6-(3-Chlor-4-fluor-phenyl)-thieno[3,2-d]pyrimidin-4-yIJ-[2-(7-fluor-2,4-dimethyl-1H-indol-3-yl)-ethyl]-amin
46. N-(3-{4-[2-(7-Fluor-2,4-dimethyl-1H-indol-3-yl)-ethylamino]-thieno[3,2-dlpyrimidin-6-yll-phenyl)-methansulfonamid
47. N-(3-{4-[2-(7-Fluor-2,4-dimethyl-1H-indol-3-yl)-ethylamino]-thieno[3,2-d]pyrimidin-6-yl}-phenyl)-acetamid
48. 6-(5-Chlor-thiophen-2-yl)-thieno[3,2-d]pyrimidin-4-yl]-[2-(7-fluor-2,4-dimethyl-1H-indol-3-yl)-ethyl]-amin
49. [6-(3,4-Difluor-phenyl)-thieno[3,2-d]pyrimidin-4-yl]-[2-(7-fluor-2,4-dimethyl-1H-indol-3-yl)-ethyl]-amin
50. [2-(7-Fluor-2,4-dimethyl-1H-indol-3-yl)-ethyl]-[6-(4-methoxy-3,5-dimethylphenyl)-thieno[3,2-d]pyrimidin-4-yl]-amin
51.[6-(3,5-Difluor-phenyl)-thieno[3,2-d]pyrimidin-4-yl]-[2-(7-fluor-2,4-dimethyl-1H-indol-3-yl)-ethyl]-amin
52.[2-(7-Fluor-2,4-dimethyl-1H-indol-3-yl)-ethyl]-[6-(4-methyl-thiophen-2-yl)-thieno[3,2-d]pyrimidin-4-yl]-amin
53.(4-{4-[2-(7-Fluor-2,4-dimethyl-1H-indol-3-yl)-ethylamino]-thieno[3,2-d]pyrimidin-6-yl}-phenyl)-acetonitril
54.[6-(2,3-Difluor-phenyl)-thieno[3,2-d]pyrimidin-4-yl]-[2-(7-fluor-2,4-dimethyl-1H-indol-3-yl)-ethyl]-amin
55.[6-(2,5-Difluor-phenyl)-thieno[3,2-d]pyrimidin-4-yl]-[2-(7-fluor-2,4-dimethyl-1H-indol-3-yl)-ethyl]-amin
56.[6-(4-Benzyloxy-2-fluor-phenyl)-thieno[3,2-d]pyrimidin-4-yl]-[2-(7-fluor-2,4-dimethyl-1H-indol-3-yl)-ethyl]-amin
57.[2-(7-Fluor-2,4-dimethyl-1H-indol-3-yl)-ethyl]-{6-[(E)-2-(4-fluor-phenyl)-vinyl]-thieno[3,2-d]pyrimidin-4-yl}-amin
58.5-{4-[2-(7-Fluor-2,4-dimethyl-1H-indol-3-yl)-ethylamino]-thieno[3,2-d]pyrimidin-6-yl}-thiophen-2-carbonitril
59.[2-(7-Fluor-2,4-dimethyl-1H-indol-3-yl)-ethyl]-[6-(2-fluor-3-methoxyphenyl)-thieno[3,2-d]pyrimidin-4-yl]-amin
60.[2-(7-Fluor-2,4-dimethyl-1H-indol-3-yl)-ethyl]-[6-(2-methoxy-pyrimidin-5-yl)-thieno[3,2-d]pyrimidin-4-yl]-amin
61.(5-{4-[2-(7-Fluor-2,4-dimethyl-1H-indol-3-yl)-ethylamino]-thieno[3,2-d]pyrimidin-6-yl}-thiophen-2-yl)-methanol
62. [6-(3-Cyclopropylmethoxy-phenyl)-thieno[3,2-d]pyrimidin-4-yl]-[2-(7-fluor-2,4-dimethyl-1H-indol-3-yl)-ethyl]-amin
63.[6-(3,5-Dimethyl-phenyl)-thieno[3,2-d]pyrimidin-4-yl]-[2-(7-fluor-2,4-dimethyl-1H-indol-3-yl)-ethyl]-amin
64.[2-(1H-Indol-3-yl)-ethyl]-[6-(3-methoxy-phenyl)-thieno[3,2-d]pyrimidin-4-yl]-amin
65.[2-(1H-Indol-3-yl)-ethyl]-(6-m-tolyl-thieno[3,2-d]pyrimidin-4-yl)-amin
66.4-{4-[2-(1H-Indol-3-yl)-ethylamino]-thieno[3,2-d]pyrimidin-6-yl}-benzonitril
67.[6-(4-Fluor-phenyl)-thieno[3,2-d]pyrimidin-4-yl]-[2-(1H-indol-3-yl)-ethyl]-amin
68.(3-{4-[2-(1H-Indol-3-yl)-ethylamino]-thieno[3,2-d]pyrimidin-6-yl}-phenyl)-methanol
69.4-{4-[2-(1H-Indol-3-yl)-ethylamino]-thieno[3,2-d]pyrimidin-6-yl}-N-methylbenzamid
70. N-(3-{4-[2-(1H-Indol-3-yl)-ethylamino]-thieno[3,2-d]pyrimidin-6-yl}-phenyl)-methansulfonamid
71. N-(3-{4-[2-(1H-Indol-3-yl)-ethylamino]-thieno[3,2-d]pyrimidin-6-yl}-phenyl)-acetamid
72.3-{4-[2-(1H-Indol-3-yl)-ethylamino]-thieno[3,2-d]pyrimidin-6-yl}-benzoesäure
73.[3-(1H-Indol-3-yl)-propyl]-[6-(3-methoxy-phenyl)-thieno[3,2-d]pyrimidin-4-yl]-amin
74.[3-(1H-Indol-3-yl)-propyl]-(6-m-tolyl-thieno[3,2-d]pyrimidin-4-yl)-amin
75.[6-(4-Fluor-phenyl)-thieno[3,2-d]pyrimidin-4-yl]-[3-(1H-indol-3-yl)-propyl]-amin
76.(3-{4-[3-(1H-Indol-3-yl)-propylamino]-thieno[3,2-d]pyrimidin-6-yl}-phenyl)-methanol
77. N-(3-{4-[3-(1H-Indol-3-yl)-propylamino]-thieno[3,2-d]pyrimidin-6-yl}-phenyl)-methansulfonamid
78. N-(3-{4-[3-(1H-Indol-3-yl)-propylamino]-thieno[3,2-d]pyrimidin-6-yl}-phenyl)-acetamid
79.3-{4-[3-(1H-Indol-3-yl)-propylamino]-thieno[3,2-d]pyrimidin-6-yl}-benzoesäure
80.[2-(2,7-Dimethyl-1H-indol-3-yl)-ethyl]-(6-m-tolyl-thieno[3,2-d]pyrimidin-4-yl)-amin
81.4-{4-[2-(2,7-Dimethyl-1H-indol-3-yl)-ethylamino]-thieno[3,2-d]pyrimidin-6-yl)-benzonitril
82.[2-(2,7-Dimethyl-1H-indol-3-yl)-ethyl]-[6-(4-fluor-phenyl)-thieno[3,2-d]pyrimidin-4-yl]-amin
83.(3-{4-[2-(2,7-Dimethyl-1H-indol-3-yl)-ethylamino]-thieno[3,2-d]pyrimidin-6-yl}-phenyl)-methanol
84.4-{4-[2-(2,7-Dimethyl-1H-indol-3-yl)-ethylamino]-thieno[3,2-d]pyrimidin-6-yl)-N-methyl-benzamid
85. N-(3-{4-[2-(2,7-Dimethyl-1H-indol-3-yl)-ethylamino]-thieno[3,2-d]pyrimidin-6-yl}-phenyl)-methansulfonamid
86. N-(3-{4-[2-(2,7-Dimethyl-1H-indol-3-yl)-ethylamino]-thieno[3,2-d]pyrimidin-6-yl}-phenyl)-acetamid
87.[2-(7-Fluor-2,4-dimethyl-1H-indol-3-yl)-ethyl]-thieno[3,2-d]pyrimidin-4-yl-amin
88. (6-Brom-thieno[3,2-d]pyrimidin-4-yl)-[2-(7-fluor-2,4-dimethyl-1H-indol-3-yl)-ethyl]-amin
89.[2-(2,7-Dimethyl-1H-indol-3-yl)-ethyl]-(6-phenyl-thieno[3,2-d]pyrimidin-4-yl)-amin
90.[2-(4-Chlor-2,7-dimethyl-1H-indol-3-yl)-ethyl]-(6-phenyl-thieno[3,2-d]pyrimidin-4-yl)-amin
91.(6-Phenyl-thieno[3,2-d]pyrimidin-4-yl)-[2-(2,4,7-trimethyl-1H-indol-3-yl)-ethyl]-amin

6. Arzneimitteln, die mindestens eine der Verbindungen der Formel I, enthalten.

7. Arzneimittel gemäß Anspruch 6 mit geeigneten Formulierungs- und Trägerstoffen.

8. Verwendung der Arzneimittel gemäß Anspruch 6 und 7, **dadurch gekennzeichnet, dass** das Arzneimittel zur Behandlung und Prophylaxe von Erkrankungen verwendet wird.

9. Verwendung der Arzneimittel gemäß Anspruch 8, zur Behandlung und Prophylaxe von Erkrankungen, die im Zusammenhang mit dem EP₂-Rezeptor stehen.

10. Verwendung gemäß Anspruch 8 zur Behandlung und Prophylaxe von Fertilitätsstörungen.

11. Verwendung gemäß Anspruch 8 zur Behandlung und Prophylaxe von Menstruationsbeschwerden.

12. Verwendung gemäß Anspruch 8 zur Behandlung und Prophylaxe von Endometriose.

13. Verwendung der Verbindungen gemäß Anspruch 1-5 zur Modulation des EP₂ Rezeptors.

14. Verwendung gemäß Anspruch 8 zur Behandlung und Prophylaxe von Schmerz.

15. Verwendung der Verbindungen gemäß Anspruch 1-5 und der Arzneimittel gemäß Anspruch 6-7 zur Fertilitätskontrolle/Kontrazeption.

16. Verwendung gemäß Anspruch 8 zur Behandlung und Prophylaxe von Osteoporose.

17. Verwendung gemäß Anspruch 8 zur Behandlung und Prophylaxe von entzündlichen Erkrankungen wie beispielsweise Morbus Crohn.

18. Verwendung gemäß Anspruch 8 zur Behandlung und Prophylaxe von Krebs.

19. Verwendung der Verbindungen der allgemeinen Formel I, gemäß den Ansprüchen 1-5, in Form eines pharmazeutischen Präparates für die enterale, parenterale, vaginale und orale Applikation.

20. Verfahren zur Herstellung der Verbindungen gemäß Anspruch 1 - 5, **dadurch gekennzeichnet, dass** die Umsetzung eines Thienopyrimidins Va bzw. Vb mit einem Amin III zu den erfindungsgemäßen Verbindungen der allgemeinen Formel I bzw. den Intermediaten der Formel II führt, und in einem weiteren Schritt die Intermediate der Formel II durch die Reaktion mit einer Boronsäure bzw. eines Boronsäureesters der allgemeinen Formel IV in einer Palladium (0)-katalysierten Reaktion zu weiteren erfindungsgemäßen Verbindungen der allgemeinen Formel I umgesetzt werden.
